(19) Europäisches Patentamt
European Patent Office
Office européen des brevets

(11) Publication number: **0 408 463 A1**

# (12) EUROPEAN PATENT APPLICATION

(21) Application number: **90402036.9**

(22) Date of filing: **13.07.90**

(51) Int. Cl.[5]: **G01N 33/52**, G01N 31/22, C12Q 1/42, C12Q 1/68, G01N 33/68, G01N 33/543, G01N 33/58, G01N 33/577, C12Q 1/58

(30) Priority: **13.07.89 EP 89402023**

(43) Date of publication of application: **16.01.91 Bulletin 91/03**

(84) Designated Contracting States: **AT BE CH DE DK ES FR GB GR IT LI LU NL SE**

(71) Applicant: **N.V. INNOGENETICS S.A.**
**Industriepark Zwijnaarde 7, Box 4**
**B-9710 Gent(BE)**

(72) Inventor: **Roelant, Chris**
**Eikenbergstraat 67**
**B-3010 Wilsele(BE)**
Inventor: **Van de Voorde, André**
**Groenstraat 22**
**B-9100 Lokeren(BE)**
Inventor: **Van Heuverswyn, Hugo**
**Colmanstraat 62**
**B-9288 Laarne(BE)**

(74) Representative: **Grosset-Fournier, Chantal Catherine et al**
**ERNEST GUTMANN-YVES PLASSERAUD S.A.,**
**67 boulevard Haussmann**
**F-75008 Paris(FR)**

## (54) Chemiluminescent compositions, chemiluminescent processes and their uses in analytical assays.

(57) The invention relates to an homogeneous hydroalcoholic chemiluminescent composition which can comprise:

- a solution containing an acridine derivative,
- a water-miscible alcohol,
- a reducing agent.

The chemiluminescent compositions of the invention are used particularly for the determination and quantification of reducing agents, pH variations in small samples, antigens or antibodies, and hybridization reactions.

EP 0 408 463 A1

# CHEMILUMINESCENT COMPOSITIONS, CHEMILUMINESCENT PROCESSES AND THEIR USES IN ANALYTICAL ASSAYS

The present invention relates to luminescent or luminometric compositions having a long-lasting light emission and high quantum efficiency, to luminescent or luminometric assays, particularly analytical methods and immunoassays, and to diagnostic kits designed to facilitate the said assays.

More particularly, the invention relates to the detection and quantification of acridine derivatives, to the detection and quantification of reducing compounds, to pH measurements in small sample volumes, and to the quantification of immunoassays and hybridization reactions.

Analytical methods based on the detection of radiation have found wide applications in the field of many of the scientific disciplines. The most popular among these methods involves the use of radioactive tracers. Although very small quantities of radioactive substances can be detected and quantified in this way, numerous hazards of manipulation as well as limitations related to the half-life of these tracers cannot be neglected.

In order to avoid the inherent disadvantages associated with the use of isotopes, several attempts have been made to replace radioisotopes by methods based on chemiluminescence reactions.

Luminescence is the emission of light produced by a chemical reaction. In contrast to fluorescence and phosphorescence (delayed fluorescence), in chemiluminescence no external energy excitation source is required for the excitation of molecules. Chemiluminescence is defined as the production of light by an exergonic chemical reaction in which molecules are left in an electronically excited state. Relaxation of these excited molecules to the ground state energy level is not a continuous process: instead, small packages of energy called "quanta" or "photons" are lost to the environment until a level of stability is obtained. Provided that the energy required to convert the lower energy state to the excited state is sufficient (40 kcal/mol to 70 kcal/mol), additional relaxation to the ground state energy level will be accompanied by the emission of light in the visible spectrum.

In chemiluminescence, increases in energy and subsequent relaxation are mostly observed with oxidation and oxygenation reactions. Several chemical compounds may become chemiluminescent upon excited single state formation. The efficiency by which these excited states are produced in chemical reactions is defined as the "quantum efficiency" or "quantum yield" of these reactions. Quantum efficiency or yield refers to a numerical value obtained by dividing the number of luminescent molecules by the number of reacting molecules. When this ratio is equal to one, the luminescence reaction is optimal (Whitehead T.P., Kricka, L.J., Couter, T.J.M. and Thorpe G.H.G., Analytical luminescence: Its potential in the clinical laboratory. Clinical Chemistry 25:1531, 1979). This is typically the case with bioluminescent reactions found in living organisms in which an enzyme catalyzes the chemiluminescent reaction. A typical example is the luciferin-luciferase reaction. Because of its high quantum efficiency, this type of reaction has been the subject of detailed investigations and is widely used in both biochemical and clinical assays (DeLuca M. and McElroy W.D., In: Methods of Enzymology (Colowick S.P. and Kaplan N.O., eds.) Academic Press, New York Vol. 57:3, 1978; Kricka L.J. and Thorpe G.H.G., Chemiluminescent and bioluminescent methods in analytical chemistry. Analyst: 1274, 1983).

Several organic compounds are known to produce chemiluminescence upon oxidation or oxygenation. One of the best known examples is luminol (5-amino-2,3-dihydro-1,4-phthalazinedione). Following oxidation, luminol is converted through a series of intermediates to an excited alpha-aminophthalate dianion that emits light during relaxation. The emission wavelength is 430 nm and the quantum efficiency is approximatively 0.01. Interestingly, it was found that luminol-dependent chemiluminescence is catalyzed by horseradish peroxidase (Arakawa H., Maeda M., Tsuji A. and Kambegawa A., Chemiluminescence enzyme immunoassay of dehydroepiandrosterone and its sulfate using peroxidase as the label. Steroids 28: 453, 1981). This enzyme is also frequently used as a label in enzyme immunoassays in which it is usually assayed by colorimetric reaction (Sternberger L.A., Hardy P.H. Jr., Cuculis J.J. and Meyer H.G.. The unlabeled antibody-enzyme method of immunochemistry. Preparation and properties of soluble antigen-antibody complex (horseradish peroxidase -antihorseradish peroxidase) and its use in identification of spirochetes. J. Histochem. Cytochem. 18:315, 1970). Taking advantage of its catalytic effect on the luminol-hydrogen peroxide reaction, attempts have been made to detect horseradish peroxidase by means of chemiluminescence. Unfortunately, chemiluminescence produced in this way is characterized by low light and therefore not suitable for routine analytical application. However, it was discovered that this type of chemiluminescence can be elevated at least 100-fold by utilizing certain "enhancer" molecules in the assay mixture. The first enhancer to be discovered was firefly luciferin. Subsequently, other more potent enhancers have been found, notably 6- hydroxybenzothiazole, p-iodophenol, p-phenylphenol and 1-bromo-2-napthol (Kricka L.J., Thorpe G.H.G. and Whitehead T.P. Enhanced luminescent or luminometric assay. European Patent Application N° 116454, 1983). Introduction of these enhancers finally led to the development of enhanced chemiluminescent immunoassays (using peroxidase as label) in conjunction with luminol and hydrogen peroxide (European Patent Application N° 87959). This system is currently commercialized under the trade name "Amerlite" by Amersham.

Although this type of luminescence analysis is easy to perform and results can be obtained rapidly, luminol-based molecules display a substantial reduction in quantum efficiency upon conjugation to other molecules (Weeks I. and Woodhead J.S. Chemiluminescence immunoassay. J. Clin. Immunoassay 7:82, 1984; Weeks, I., Beheshti I., McCapra F. et al. Acridinium esters as high specific labels in immunoassays. Clin. Chem. 29: 1474, 1983). In this way, the use of the "Amerlite" system is limited to the detection of horseradish peroxidase.

Recently, increased interest has been shown in the use of acridine-based molecules for the development of luminescent assays. In contrast to luminol-based molecules, acridine-derived molecules can easily be coupled to proteins without any reduction in quantum efficiency due to differences in the nature of the chemiluminescence reaction (Weeks, I., Behesti I., McCapra F. et al. Acridinium esters as high specific labels in immunoassays. Clin. Chem. 29: 1474, 1983). Under alkaline conditions it has been shown that a typical acridine salt-like bis-N-methyl-acridine dinitrate (lucigenin) is oxidized by hydrogen peroxide to two molecules of N-methylacridone, one of which is formed in the excited state. Relaxation of this excited state is accompanied by the emission of light at 470 nm (Allen R.C. In Liquid Scintillation Counting: Recent Applications and Developments, Vol. 2, Peng C.T., Horrocks D.L. and Alpen E.L., (Eds) Academic Press, New York:37:1980).

Based on the same principle, acridine based esters have been synthesized which emit light upon concerted multiple-bond cleavage via a dioxetane intermediate to yield vibronically excited molecules of N-methylacridone (McCapra F., Chemiluminescence of organic compounds. Prog. Org. Chem. 8: 231, 1977). It is important to note that the excited product molecule is thereby dissociated from the original molecules prior to the emission of photons. In this way, any structural modifications concerning substitutions at the alcoholic part of the ester do not influence the final light output (Weeks, I., Behesti I., McCapra F. et al. Acridinium esters as high specific labels in immunoassays. Clin. Chem. 29: 1474, 1983).

Others have synthesized substrates with stabilized dioxetane compounds which are cleaved upon enzymatic action, yielding the free dioxetane intermediate. In this way, the rate of light emission is dependent on the enzymatic action (European Patent Application No. 254051).

Coupling of aryl acridinium esters to N-succinimidyl esters has been successfully used for the labeling of antibodies with minimal loss of their immunological activity (European Patent Application No. 263657). As such the method is applied in several assays which are currently being marketed under the trade name "Magic Lite" by Ciba Corning.

Neither the "Amerlite system" nor the "Magic Lite" system have found wide application in routine analysis, mainly due to the fact that in those systems, the light emission upon excitation is invariably short-lived: only a "flash of light" is produced with acridinium ester molecules ("Magic Lite" system) and the signal of horseradish peroxidase-catalyzed luminol-dependent chemiluminescence ("Amerlite" system), even in the presence of an enhancer, may decrease by 30% over a 30-minute period (Edwards J.C. Development and performance of the Amerlite system. Enhanced luminescence: a practical immunoassay system. In: Proceedings of a Workshop, Minster Lovell, UK, 1985. The Medicine Publishing Foundation. Symposium Series 18).

Finally, because of their specific embodiments, both types of assays require the use of special instrumentation, such as luminometers, for the detection of the signal.

The present invention, on the one hand, is able to overcome the problems inherent to the prior art chemiluminescent systems and, on the other hand, offers a wide scope of applications in analytical processes.

In one of its aspects, the invention provides a chemiluminescent composition which has a long lasting emission of the light signal and which has a high quantum efficiency.

In another of its aspects, the invention provides a chemiluminescent composition in which the aqueous environment is reduced, which offers the advantage of sample reduction.

In another of its aspects, the invention provides a chemiluminescent composition in which no enzyme is necessary, which avoids stability and storage problems.

In another of its aspects, the invention provides a chemiluminescent composition which enables detection of pH variations and measurement of pH, particularly in small sample volumes.

In another of its aspects, the invention provides quantitative and/or qualitative analysis of various chemical or biochemical compounds.

In another of its aspects, the invention provides a chemiluminescent composition which allows the detection and quantification of acridine derivatives, particularly in small sample volumes.

In another of its aspects, the invention provides a chemiluminescent composition which allows the detection and quantification of reducing agents, particularly in small sample volumes.

In another of its aspects, the invention provides a chemiluminescent composition which allows the quantification of immunoassays, and hybridization reactions, particularly in small sample volumes.

In another of its aspects, the invention provides a chemiluminescent composition which allows determination and quantification of enzymatic activity such as urease, alkaline phosphatase or peroxydase activity.

In another of its aspects, the invention provides a chemiluminescent composition enabling performance of chemiluminescent assays in which the detection of the light signal does not require the use of special instrumentation.

All these aspects have been obtained by an homogeneous hydroalcoholic chemiluminescent composition comprising:

- a solution containing an acridine derivative, said solution being preferably aqueous,
- a water-miscible alcohol,
- a reducing agent, which can be either present or absent, depending on the appropriate conditions of pH, provided that the alcohol is different from an alcohol derived from an acridine derivative and is different from acridanol and is different from an alcohol derived from an acridanol derivative, which might be formed during the chemiluminescent reaction.

The acridine derivative can be either water-soluble or alcohol-soluble, or can be solubilized in the homogeneous hydroalcoholic composition.

The expression "chemiluminescent composition" means that, when all the components listed above (i.e., acridine derivative, a water-miscible alcohol and, possibly, a reducing agent) are mixed, light is emitted due to the chemiluminescent reaction between these components, the emission of light being likely to result from the acridine derivative which generates an intermediate molecule liable to relax from an excited state to a ground state.

The expression "homogeneous hydroalcoholic chemiluminescent composition" means that the composition comprises alcohol and an aqueous solution, the alcohol being water-miscible in the aqueous solution, which results in a homogeneous aqueous phase.

The expression "water-miscible alcohol" means that either the alcohol is water-soluble or if is not water-soluble, it can form a homogeneous suspension in water, that is sufficiently stable for the chemiluminescent reaction to take place.

In case of a non water-soluble alcohol, the suspension of alcohol in water must be stable enough, for the chemiluminescence to take place, and preferably over a period long enough for the chemiluminescence reaction to last. The suspension of alcohol in water is more preferably stable over a period at least as long as the light emission, so that there is no interruption of the light emission due to instability of the chemiluminescence composition.

When the alcohol used is not water-soluble, the homogeneous hydroalcoholic solution is obtained by shaking, for example sonicating, the aqueous solution containing the alcohol.

In such a case, the stability of the homogeneous hydroalcoholic solution can be maintained by means of stabilizing agents, such as emulsifiers, as long as said stabilizing agent does not interfere with the molecular structure of the acridine derivative, and especially with the intermediate excited compound which generates light emission.

The alcohol is a primary, secondary, or tertiary aliphatic alcohol, heterocyclic alcohol, or a polyalcohol.

The definition of alcohol excludes sugars, but includes mercapto alcohols.

In this form, the present invention features:

- high quantum efficiency of the chemiluminescent reaction and optimized long-lasting light emission so that no special instrumentation is required for the injection of reagents positioned in front of a photomultiplier, thus reducing the complexity of the assay to that currently used in enzyme-linked immunosorbent assays,
- the assay sensitivity, which is equal or better than that usually obtained with radioimmunoassays.

The chemiluminescent composition of the invention provides a light emission which lasts for more than about 50 minutes and which can last for as long as about 10 days. The light signal resulting from the chemiluminescent composition of the invention remains substantially constant at the maximum value reached during the whole period of the light emission.

The quantum efficiency of the chemiluminescent composition of the invention is higher than the quantum efficiency of acridine derivative used in a classical chemiluminescent reaction and is above 0.005, particularly above 0.01, and especially above 0.05.

The intensity of the light signal varies from about 100 to about 6 x $10^6$ (expressed in counts per minute (c.p.m.)).

The chemiluminescent composition of the invention comprises:

- an alcohol in the absence of a reducing agent provided that the pH of the aqueous phase is appropriate so that the chemiluminescent reaction can take place,
- or comprises an alcohol in the presence of a reducing agent.

When the composition of the invention does not include a reducing agent, the pH of the aqueous solution must be such that the alcohol itself can provoke the chemiluminescent reaction. The pH of the aqueous solution can be adjusted for the alcohol to provoke the chemiluminescent reaction by the addition of a base.

There are no restrictions as to the base as long as it does not interfere with the molecular structure of the acridine derivative and especially with the intermediate excited compound which generates light emission.

The amount of alcohol necessary in the chemiluminescent composition depends on the nature of the alcohol and on the pH of the aqueous solution and is a decreasing function of the pH - which means that the higher the pH, the less alcohol that is necessary.

When the pH of the aqueous solution is such that the alcohol is not appropriate to provoke the chemiluminescent reaction, a reducing agent is then necessary.

When there is a reducing agent in the composition of the invention, then said reducing agent is part of the aqueous solution.

In this case, the chemiluminescent composition of the invention can be defined as comprising:

- a solution containing an acridine derivative and a reducing agent, the solution being preferably aqueous,
- a water-miscible alcohol.

According to an advantageous embodiment, the chemiluminescent composition of the invention comprises a reducing agent, in addition to the acridine derivative and the alcohol, even if the pH of the aqueous solution is such that the alcohol can provoke the chemiluminescent reaction by itself, with such a composition presenting the advantage of increased sensitivity.

According to another advantageous embodiment, the chemiluminescent composition of the invention comprises a base to increase the pH in addition to the reducing agent, the alcohol and the acridine derivative. Such compositions present the advantage of increasing the sensitivity of the chemiluminescent reaction.

Depending on the process or assay in which the chemiluminescent compositions of the invention may be involved, the pH of the aqueous solution may advantageously be in a higher pH range without the use of a reducing agent, or in a lower pH range with the use of a reducing agent.

The aqueous solution of the acridine derivative can be constituted by a biological sample in which an acridine derivative is dissolved. In such a case, the pH of the aqueous solution is such that the chemiluminescence of the invention must comprise a reducing agent.

The biological samples which can be involved in the invention are for instance: blood, cerebrospinal liquid, single cells, sputum.

According to another embodiment of the invention, the chemiluminescent composition comprises an aqueous buffer, such as phosphate buffer, Tris buffer, acetate buffer, acetate buffer, diethanolamine buffer, barbital buffer etc...

Appropriate buffers used are the ones which do not interfere with the molecular structure of the acridine derivative, especially with the intermediate compound responsible for the chemiluminescent reaction.

The buffer must also be appropriate so as not to interfere with the assay or process in which the chemiluminescent composition of the invention is involved.

For instance, if the chemiluminescent composition is used to detect and quantify the enzymatic activity of alkaline phosphatase, it is appropriate not to use a phosphate buffer.

This aqueous buffer is not essential for the chemiluminescent reaction per se, but may be useful in some applications, such as analytical methods in which the chemiluminescent reaction generated by the chemiluminescent composition of the invention is involved.

In such a case, the presence of the buffer enables the control of the pH at which the activity of the enzyme is optimal.

In the chemiluminescent composition of the invention, the acridine derivative is either water-soluble or alcohol-soluble or can be solubilized in the homogeneous hydroalcoholic composition, and must be able to generate an intermediate molecule liable to relax from an excited state to a ground state and to return to an excited state, in a cyclic manner.

For example, the acridine derivative has the following formula:

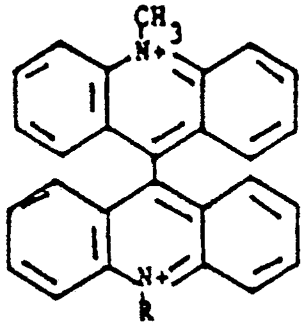

$2X^-$ or $X^{2-}$

wherein:

$2X^-$ or $X^{2-}$ represents a negatively charged counterion (so that the positive charge of the above molecule is compensated by the negative charge of X) or a nitrate, sulfate, phosphate, chlorate counterion,

R represents an aliphatic chain of 1 to 6 carbon atoms, particularly 1 to 4 carbon atoms, and more particularly $CH_3$,

with at least one of the phenyl rings being optionally substituted, provided that the acridine derivative is either water-soluble or alcohol-soluble or can be solubilized in the homogeneous hydroalcoholic composition, preferably water-soluble,

said acridine derivative being preferably lucigenin,

or

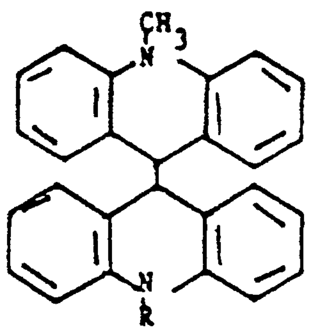

wherein R has the above-mentioned meaning,

with at least one of the phenyl rings being optionally substituted, provided that the acridine derivative is either water-soluble or alcohol-soluble or can be solubilized in the homogeneous hydroalcoholic composition, preferably water-soluble,

or

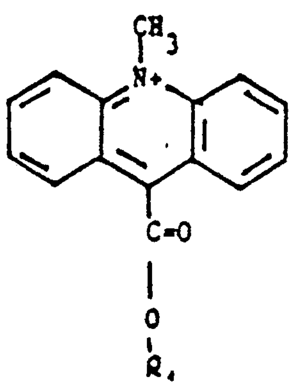

$Y^-$

wherein $Y^-$ represents a negatively charged counterion (so that the negative charge of Y is compensated by the positive charge of the above molecule) such as $FSO_3^-$, $NO_3^-$,

$R_1$ represents

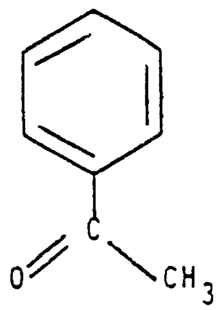 , 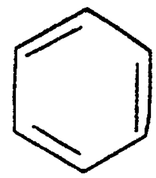 , 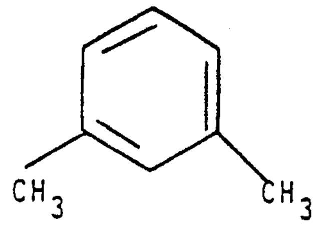

with at least one of the phenyl rings being liable to be substituted provided that the acridine derivative is either water-soluble or alcohol-soluble or can be solubilized in the homogeneous hydroalcoholic composition, preferably water-soluble,
or

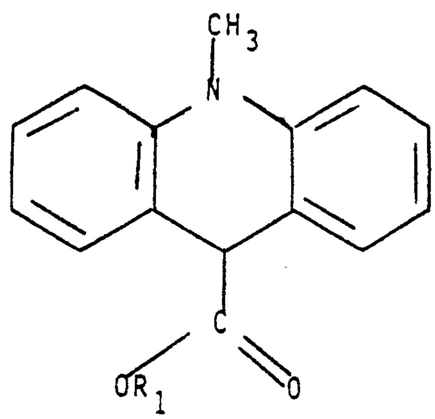

wherein $R_1$ has the above-mentioned meaning,
with at least one of the phenyl rings being liable to be substituted provided that the acridine derivative is either water-soluble or alcohol-soluble or can be solubilized in the homogeneous hydroalcoholic composition, preferably water-soluble.

Lucigenin is a preferred acridine derivative.

As to the alcohol, it is preferably different from a mercapto alcohol.

In preferred compositions of the invention, the water-miscible alcohol is primary, secondary or tertiary alcohol of 1 to 6, particularly 1 to 4 carbon atoms, particularly methanol, ethanol, n-propanol, isopropanol or butanol.

When methanol, ethanol or propanol is used, the alcohol is preferably qualified as being water-soluble, whereas when butanol is used, it is qualified as being water-miscible.

Advantageous water-soluble alcohols are polyvinyl alcohol and the alcohol commercialized under the trademark Triton X100.

According to the process in which the chemiluminescent composition of the invention is involved, propanol may be advantageous, when the pH of the aqueous solution is as low as about 3. As a result, it is consequently not necessary to use a reducing agent, particularly when the volume of alcohol represents 90% of the total volume of the composition.

An advantageous range of pH of the aqueous solution in the chemiluminescent composition of the invention is from about 0 to about 14, particularly from about 4 to about 8, especially from about 6 to about 7.6.

Such pH ranges are found in biological samples in which such chemiluminescent assays might be involved.

The reducing agent must be such that it does not interfere with the molecular structure of the acridine derivative and especially with the intermediate compound(s) responsible for the chemiluminescent reaction.

The reducing agent has preferably an oxido-reduction potential of at least 0.2V, preferably at least about 0.3V.

For instance, appropriate reducing agents are ascorbic acid, mannose, glucose, NADH or NADPH, or hydroquinone.

According to another embodiment of the invention, the chemiluminescent composition comprises an enzyme, in addition to the aqueous solution containing an acridine derivative, possibly a reducing agent, possibly a base and possibly a buffer.

The enzyme can be urease, or alkaline phosphatase.

According to another embodiment, the chemiluminescent composition of the invention can comprise, in addition to the enzyme, a substrate for the enzyme.

According to another embodiment, the chemiluminescent composition of the invention can contain a substance which is able to increase the pH when it is contacted with the element to be determined and quantified.

For instance, the chemiluminescent composition of the invention can comprise ninhydrin which becomes a pH-increasing substance to be determined by liberation of $NH_3$, when placed in contact with an amino acid.

Such chemiluminescent compositions of the invention are useful for analytical processes.

The different elements of the chemiluminescent composition of the invention can be present at any time at a given concentration or can be generated in their active form in situ .

In the case of the acridine derivative, it can be liberated in situ , for example from an encapsulated form, such as a polymeric encapsulation, for instance liposomes, niosomes, provided that the encapsulation does not interfere with the chemiluminescent reaction.

In the case of the alcohol, the alcohol may be produced in situ by chemical reactions.

In the case of the reducing agent, a pH-dependent agent can be used such as hydroquinone which becomes a reductant when the pH of the aqueous solution reaches 7.8 and goes above this value.

An advantageous chemiluminescent composition of the invention is the following:

- acridine derivative : from about 0.01% to about 99.99% (in volume), particularly from about 10% to about 90% of a concentration of about $10^{-4}$ to about $10^{-10}$ M/l, particularly from about $10^{-4}$ to about $10^{-6}$ M/l;
- alcohol (pure alcohol of commercial source): from about 0.5% to about 99.5% (in volume), particularly from about 10% to about 99.5%, all these percentages are expressed in volumes and the concentrations are expressed with respect to the total volume of the chemiluminescent composition of the invention.

Another preferred chemiluminescent composition of the invention comprises:

- acridine derivative: from about 0.01% to about 99.99%, particularly from about 10% to about 90% (in volume), of à concentration range of about $10^{-4}$ to about $10^{-10}$ M/l;
- alcohol (of pure alcohol): from about 10% to about 99.5% (in volume), particularly from about 10% to about 90%;
- reducing agent: from about 0.1% to about 99.9% (in volume) of a concentration of about 100 ng/ml to about 10 mg/ml.

The temperature of the chemiluminescent composition is preferably moderate, ranging from about 10°C to about 50°C, preferably room temperature. It is preferable that the temperature of the chemiluminescent composition of the invention is below the boiling point of alcohol to avoid its evaporation.

To prepare the chemiluminescent compositions of the invention, the different components are assembled together, in any order, that is to say the aqueous solution, preferably buffered, of acridine derivative (and of the possible reducing agent and/or the possible base) and the alcohol are mixed together, without any preferred order of addition of the various components.

However, when the chemiluminescent composition of the invention is used in an assay involving an enzymatic reaction, it may be advantageous to add the alcohol to the solution, preferably aqueous (containing an acridine derivative, an enzyme, and possibly a reducing agent, and a buffer), when the enzymatic reaction has reached the appropriate desired state or is terminated because the presence of the alcohol may halt the enzymatic reaction.

The chemiluminescent composition of the invention can be used under conditions such that light emission can be made dependent on:

1. the amount of the acridine derivative, if the reducing agent and the alcohol are in excess,
2. the amount of the reducing agent, if the acridine derivative and the alcohol are in excess,
3. the nature and the concentration of the alcohol, if the acridine derivative and the reducing agent are in excess,
4. the pH of the aqueous buffer solution.

The invention also relates to luminescent or luminometric assays wherein the chemiluminescent reaction is based on the chemiluminescent composition.

The invention also relates to a process for determining the concentration of acridine derivative in a

solution, preferably aqueous, wherein the concentration of the reducing agent is constant, with the concentration of the alcohol is constant and the pH is constant, the rate of the light produced in the chemiluminescent reaction being a function of the concentration of the acridine derivative.

For the quantitative detection of acridine derivative, the chemiluminescent composition of the invention must be such that the alcohol is in excess with respect to the acridine derivative, and if there is a reducing agent, the reducing agent is in excess with respect to the acridine derivative.

The excess of alcohol with respect to the acridine derivative means that there is at least 90% (in volume) of alcohol with respect to the total volume of the composition.

The excess of the reducing agent with respect to the acridine derivative means that there is at least 1 mg/ml of reducing agent.

The quantitative determination of acridine is advantageously carried out when alcohol represents a volume of about 99% of the total volume of the chemiluminescent composition, and the volume of the aqueous solution of acridine (of a concentration range of about $10^{-3}$ M to about $10^{-7}$ M) represents about 1% of the total volume of the chemiluminescent composition.

When the chemiluminescent composition of the invention comprises a reducing agent, it can represent a volume percentage of about 1% of the total volume of the chemiluminescent composition (for a concentration range of the reducing agent of about 10 µg/ml to about 1 mg/ml).

When the pH of the composition is, for instance, above 9, the excess of alcohol with respect to acridine can be such that the alcohol/ acridine volumetric ratio is from about 10% / 90% to about 99% / 1%.

When the pH of the composition is, for instance, in the range of 5-7, the excess of alcohol with respect to acridine can be such that the alcohol/acridine volumetric ratio is from about 60% / 40% to about 99% / 1%.

The quantification of acridine might be useful for the determination of an antigen or an antibody, by appropriate coupling of the antigen or of the antibody to be determined to an acridine derivative.

The acridine to be quantified can be enclosed in an encapsulated form, such as in a polymer, for instance in liposomes or niosomes, provided that the encapsulated form does not interfere with the chemiluminescent reaction, with the said encapsulated form being itself used as a label for the quantification of an antigen or of an antibody, or of a nucleic acid sequence.

The invention also relates to a process for determining the concentration of the reducing agent contained in an aqueous solution, wherein the concentration of the acridine derivative, the concentration of the alcohol and the pH are kept constant, with the rate of the light produced in the chemiluminescent reaction being a function of the concentration of the reducing agent.

For the quantitative determination of a reducing agent, the chemiluminescent composition of the invention must be such that the acridine derivative is in excess with respect to the reducing agent, and the alcohol is in excess with respect to the reducing agent.

The excess of acridine derivative with respect to the reducing agent means that there is a $10^{-3}$M to $10^{-5}$M concentration range of acridine.

The excess of alcohol with respect to the reducing agent means that there is at least 60% (in volume) of the alcohol, depending on the nature of the alcohol.

For the quantification of a reducing agent, an appropriate composition which corresponds to an excess of acridine derivative and an excess of alcohol with respect to the reducing agent is as follows:

- about 1% of acridine derivative (in volume) of concentration of about $10^{-4}$ M,
- about 90% of alcohol (in volume), the volume of the reducing agent (of a concentration range of about 1 pg/ml to about 1 mg/ml), representing about 9% of the total volume of the chemiluminescent composition.

Acridine derivative-dependent enhanced chemiluminescence based on changes in reducing capacity of the chemiluminescent phase, preferably aqueous phase, can be used for the detection and quantification of any label or substance of interest which is reducing by itself or may become reducing by a chemical reaction.

The invention also relates to a process for determining the pH, or the variations of pH of aqueous solutions, particularly of minute amounts of solute, for example of less than about 10 µl, particularly from about 0.1 µl to about 1 µl, wherein the concentration of the acridine derivative is constant, the concentration of the alcohol is constant and the concentration of the reducing agent is constant, the rate of the light produced in the chemiluminescent reaction being a function of the pH.

In this embodiment of the invention, pH variations can be generated in situ either in a stoichiometric way or in a time-dependent manner.

According to a preferred embodiment of the invention, pH increases of aqueous solutions, such as biological samples, can be measured.

For instance, the pH can be increased by introducing fixed amounts of pH-increasing substances, such

as basic ions, for instance OH-, CN-, polybasic ions or highly basic molecule such as spermine, spermidine, and cadaverine. In this way, the pH is increased in a stoichiometric way.

The pH can also be increased by an enzymatic reaction, such as that resulting from the addition of an enzyme generating ions, for instance the addition of urease which generates $NH_4^+$ ions.

In this way, the pH is increased in a time-dependent way.

If the chemiluminescent reaction takes place before the end of the enzymatic reaction in which urease generates $NH_4^+$ ions, then the increase of the pH is a time-dependent reaction.

If the chemiluminescent reaction takes place once the above-mentioned enzymatic reaction is terminated, then the pH is increased in an independent time fashion, i.e. in a stoichiometric way.

For determining the pH increase resulting from enzymatic reactions, the pH of the aqueous solution before the variations (initial pH), is advantageously the pH at which the enzyme which is responsible for the increase of pH has its maximum activity.

For instance, when urease is used, the initial pH is advantageously of 6.5 in acetate buffer and 7.5 in phosphate buffer.

For the determination of the pH increase resulting from a pH-increasing substance not generated from an enzymatic reaction, the initial pH can have any value. But, in such a case, it might be necessary to carry out a control determination without the pH-increasing substance.

The determination of the pH variation of an aqueous solution is advantageously carried out in such conditions that before the addition of the pH increasing substance, no chemiluminescent reaction can take place.

According to the invention, pH decreases in aqueous solution can also be measured.

The determination of pH variations in small volumes might be useful in clinical laboratories and surgical units, for instance to determine pH variations of biological samples such as blood serum during operations.

The invention also relates to a process wherein the concentration of the acridine compounds and the concentration of the water-soluble compounds are constant, and a constant amount of reducing agent is generated upon reaching or surpassing a predetermined pH either in a stoichiometric or time dependent fashion.

The invention relates to a process for quantifying pH-increasing substances, comprising the introduction of a pH-increasing substance, such as basic, or polybasic ions, or enzymes generating such ions such as urease generating $NH_4^+$ into a solution of acridine derivative and of alcohol; the rate of light produced is a function of the pH increasing substance.

According to an advantageous embodiment, the solution of acridine derivative and alcohol contains a fixed amount of an agent liable to acquire appropriate reducing properties above a determined pH such as hydroquinone which becomes reductant at a pH 7.8.

The advantage of the use of such a system is to rule out any light emission provided that a determined pH has been established.

The determination of pH-increasing substances can be used for quality control of pharmaceutical preparations or for the detection of any label capable of inducing a predetermined shift in pH and for the determination of an antigen, an antibody, or of a nucleic acid sequence.

For the determination of a substance involving the use of urease, urease can be enclosed in an encapsulated form, such as by polymeric encapsulation, for example in liposomes or niosomes, provided the encapsulated form does not interfere with the chemiluminescent reaction, with the said encapsulated form being liable to be used as the label for the substance to be determined.

The invention also relates to a process for quantifying antigens or antibodies, and involving the chemiluminescent composition of the invention.

Such a process can comprise the introduction of a solution containing the antigen to be quantified bound to a pH-increasing substance, or an antibody to be quantified bound to a pH-increasing substance into a solution of acridine derivative and of alcohol, advantageously in the presence of a reducing agent, with the rate of the light produced being a function of the pH-increasing substance from which the quantification of antigen or antibody can be deduced by comparison with calibration curves.

According to an advantageous embodiment, the solution of acridine derivative and alcohol contains a fixed amount of an agent liable to acquire reducing properties above a determined pH.

The advantage of the use of such a system is to rule out any light emission provided a determined pH has been established.

According to another preferred process of the invention, the antigen or antibody to be determined acts as a pH-dependent reducing system, and the chemiluminescent composition used for the assay contains a pH-increasing substance.

Such antigen or antibody acting as pH dependent reducing systems include an immobilized antibody

(for instance fixed on a solid support) capturing an antigen such as a bacteria or other eukaryotic cells or a part thereof, which themselves are stimulated to produce a reducing agent, so that there is a change in the reducing capacity of the aqueous phase which contains them.

According to another preferred process of the invention, the antigen or antibody to be determined is bound to a pH-dependent reducing system, and the chemiluminescent composition of the invention used for the assay contains a pH-increasing system.

According to another advantageous embodiment of the invention, the antigen or antibody to be quantified can be bound to a reducing agent and the quantification of the antigen or of the antibody results from the quantification of the reducing agent.

According to another preferred process of the invention, the antigen or antibody to be quantified can be bound to an acridine derivative, and the quantification of the antigen or of the antibody results from the quantification of the acridine derivative.

The invention also relates to the quantification of a hybridization reaction of a probe with a nucleic acid sequence to be detected, provided the hybridizable probe is labeled with a pH-increasing substance, advantageously in the presence of a reducing agent particularly a pH-dependent reducing agent, such as described above, or with a moiety that can be recognized by an antibody labeled with a pH-increasing substance, advantageously in the presence of a reducing agent, particularly a pH-dependent reducing agent.

The invention also relates to a method for detecting and quantifying amino acids involving the chemiluminescent composition of the invention, based on the determination of a pH-increasing substance such as ninhydrin.

Ninhydrin is a pH-increasing substance when brought into contact with an amino acid, due to the liberation of $NH_3$.

Such a process related to the invention for detecting and quantifying amino acids comprises addition of ninhydrin into a chemiluminescent composition of the invention containing the amino acid to be detected and quantified. The amount of the ninhydrin which has reacted with the amino acid to be detected and quantified can be determined and, consequently the amount of the amino acid can be detected and quantified by comparison with calibration curves.

The invention also relates to a process for measuring alkaline phosphatase activity on a substrate containing phosphate group(s), wherein the chemiluminescent composition comprises a solution of an acridine based moiety, preferably in buffered solution, and mannose, and in the presence of an alcohol. The rate of light produced in the chemiluminescent reaction is a function of phosphate groups released from the substrate by the action of alkaline phosphatase.

When the chemiluminescent composition of the invention is used for the determination of the activity of alkaline phosphatase, the buffer is advantageously diethanolamine buffer and not a phosphate buffer.

The substrate containing phosphate group(s) is any component containing phosphate group(s), insofar as it does not interfere with the molecular structure of the acridine derivative, for instance ATP, 1,2-glycerophosphate, p-nitrophenylphosphate, β-glycerophosphate, phosphocreatine, indolylphosphate.

The pH for the measurement of alkaline phosphatase according to the invention is advantageously one at which the alkaline phosphatase has its maximum enzymatic activity, advantageously about 9.8.

The determination of alkaline phosphatase activity using the chemiluminescent composition of the invention can be used for the quantitative determination of an antigen or an antibody. In that respect the antigen or antibody to be determined can be bound to the enzyme, which is reacted with a substrate with the rate of light produced in the chemiluminescent reaction being a function of turnover.

For the quantification of an antigen or of an antibody, alkaline phosphatase can be enclosed in an encapsulated form, such as by a polymeric encapsulation, for example in liposomes or niosomes, provided that the encapsulated form does not interfere with the chemiluminescent reaction, with the said encapsulated form being itself used as a label for the quantification of an antigen, an antibody or a nucleic acid sequence.

In another embodiment, the antigen or antibody to be quantified can be bound to a substrate containing phosphate group(s) which is reacted with alkaline phosphatase, the rate of light produced in the chemiluminescent reaction being a function of phosphate groups of the substrate released by the action of alkaline phosphatase.

The determination of alkaline phosphatase activity using the chemiluminescent composition of the invention can be used for quantitative and qualitative detection of a hybridization reaction of a probe with a nucleic acid sequence to be detected.

According to an embodiment, the probe is prelabeled with a label, and an alkaline phosphatase-labeled antibody (monoclonal or polyclonal) against the said label is used for the enzymatic reaction to take place

between the alkaline phosphatase and a substrate containing phosphate group(s), the rate of the light produced in the chemiluminescent reaction being a function of phosphate groups released by the alkaline phosphatase.

The invention also relates to kits comprising the components necessary to carry out the assay in which the chemiluminescent composition of the invention is involved.

For the quantification of acridine derivatives, a kit of the invention comprises:

- an alcohol,
- possibly a buffer,
- a reducing agent,
- possibly a base for the pH of the composition to be adjusted to a constant value.

For the quantification of a reducing agent, a kit of the invention comprises:

- an acridine derivative,
- possibly a buffer,
- possibly a base for the pH of the composition to be adjusted to a constant value,
- an alcohol.

For the quantification of pH variation, a kit of the invention comprises:

- an acridine derivative,
- possibly a buffer,
- possibly a reducing agent,
- an alcohol.

For the quantification of pH-increasing substances, a kit of the invention comprises:

- an acridine derivative,
- possibly a buffer,
- possibly a reducing agent,
- an alcohol.

For the quantification of an antigen or of an antibody, a kit of the invention comprises:

- an acridine derivative,
- an alcohol,
- possibly a buffer,
- a reducing agent, preferably a pH-dependent reducing system, liable to be coupled to the antigen or antibody to be determined,
- a pH-increasing system, such as an enzyme,
- possibly a substrate for the enzyme.

Another kit of the invention for the quantification of an antigen or an antibody comprises:

- an acridine derivative,
- an alcohol,
- possibly a buffer,
- a pH-increasing system, such as an enzyme, for instance urease, liable to be coupled to the antigen or antibody to be determined,
- possibly a substrate for the enzyme,
- possibly a reducing agent, preferably a pH-dependent reducing system such as hydroquinone.

For the quantification of nucleic acid sequences, a kit of the invention comprises:

- an acridine derivative,
- an alcohol,
- possibly a buffer,
- a reducing agent, preferably a pH-dependent reducing system, liable to be coupled to the nucleic acid sequence to be determined,
- a pH-increasing system, such as an enzyme,
- possibly a substrate for the enzyme.

Another kit of the invention for the quantification of nucleic acid sequences comprises:

- an acridine derivative,
- an alcohol,
- possibly a buffer,
- a pH-increasing system, such as an enzyme, for instance urease, liable to be coupled to the nucleic acid sequence to be determined,
- possibly a substrate for the enzyme,
- possibly a reducing agent, preferably a pH-dependent reducing system such as hydroquinone.

For the quantification of amino acids, a kit of the invention comprises:

- an acridine derivative,
- an alcohol,
- possibly a buffer,
- a substance which is pH-increasing, when reacted with the amino acid to be determined, such as ninhydrin,
- possibly a reducing agent, preferably a pH-dependent reducing agent, such as hydroquinone.

For the quantification of an antigen, a kit of the invention comprises:

- an acridine derivative,
- an alcohol,
- possibly a buffer,
- a polyclonal, preferably monoclonal antibody against the antigen to be determined,
- means for detecting the antibody directed to the antigen to be determined, liable to be coupled to urease, the said means being for instance an antibody against the antibody directed against the antigen to be determined, coupled to urease,
- a substrate for urease.

A kit of the invention for quantifying an antigen or an antibody comprises:

- an acridine derivative,
- an alcohol
- a reductant, preferably mannose
- possibly a buffer,
- alkaline phosphatase enzyme liable to be bound to the antigen or antibody to be determined,
- a substrate for alkaline phosphatase.

An advantageous kit of the invention for quantifying an antigen comprises:

- an acridine derivative,
- an alcohol
- a reductant, preferably mannose
- possibly a buffer,
- an antibody to the antigen to be determined, coupled to alkaline phosphatase,
- a substrate for the alkaline phosphatase such as p-nitrophenylphosphate.

An advantageous kit of the invention for quantifying a nucleic acid sequence comprises:

- an acridine derivative,
- an alcohol,
- possibly a buffer,
- a reductant, preferably mannose,
- a prelabeled probe, such as digoxigenin prelabeled probe,
- an antibody directed against the label of the probe, liable to be bound to alkaline phosphatase, with the said antibody being for instance an alkaline phosphatase-labeled anti-digoxigenin antibody,
- a substrate for the alkaline phosphatase such as p-nitrophenylphosphate.

The invention relates particularly to a kit for the detection and quantification of any antigen (or antibody) present in a test medium such as body fluids and cell supernatants and which comprises:

- means for the fixation of an antibody (or antigen) liable to form an immunological complex with the antigen (or antibody) to be determined,
- a monoclonal or polyclonal antibody forming a complex with a pH-increasing substance or a pH-increasing enzyme such as urease or a phosphate-generating enzyme such as alkaline phosphatase, with the said antibody being liable to form a specific bond with the antigen (or antibody) to be determined,
- a chemiluminescent composition of the invention.

The invention relates to an advantageous kit for the detection and quantification of any antigen (or antibody) present in a test medium such as body fluids and cell supernatants and which comprises:

- a solid support preferably microwells, plastic luminometer vials or protein-binding membranes, to which proteins (i.e. antibody when the substance to be determined is an antigen, or antigen when the substance to be determined is an antibody) have been bound, covalently or non-covalently, with the said proteins being able to form an immunological complex with the antigen (or antibody) to be determined in the test medium,
- a monoclonal or pclyclonal antibody forming a complex with a pH-increasing substance or a pH-increasing enzyme such as urease or a phosphate- generating enzyme such as alkaline phosphatase and liable to form a specific bond with the antigen (or antibody) to be determined,
- a chemiluminescent composition of the invention.

The invention relates more particularly to a kit for detecting and quantifying a particular target nucleic acid sequence in a test medium, comprising in one or more containers:

- a first single-stranded nucleotide probe capable of hybridizing with a nucleotide sequence present in the target nucleic acid sequence to be detected, with the said first probe being fixed on fixation means,
- a second chemically or enzymatically labeled nucleotide probe capable of hybridizing with a nucleotide sequence present in the target nucleic acid sequence, with the said second probe being unable to hybridize with the first probe fixed on fixation means,
- means for detecting the label of the second probe, with the said means being conjugated to urease or alkaline phosphatase or acridine-loaded liposomes,
- a chemiluminescent composition of the invention.

The invention relates more particularly to a kit for detecting and quantifying a particular target nucleic acid sequence in a test medium, comprising in one or more containers:
- a first polynucleotide probe comprising a single-stranded nucleotide sequences capable of hybridizing with the target nucleic acid sequence to be detected, with the said first probe being fixed to a solid support preferably in the form of strips, microtiter plates or plastic luminometer vials,
- a second chemically or enzymatically labeled nucleotide probe (for instance labeled with biotin, digoxigenin) capable of hybridizing with a nucleotide sequence present in the target nucleic acid sequence, with the said second probe being not able to hybridize with the first probe fixed to the solid support,
- a protein or any other substance which can be attached to the said label, the said protein or any other substance being for example avidin or anti-digoxigenin antibodies and being conjugated to urease or alkaline phosphatase or acridine-loaded carriers,
- an appropriate chemiluminescent composition of the invention.

The various aspects of the invention are illustrated by the following examples which are not a limitation of the invention.

EXAMPLE I: Extended chemiluminescence emission:

Long-lasting chemiluminescence according to the present invention can be produced as shown in Figure 1, in which the intensity of the emitted light signal (in c.p.m. in coincidence mode) is plotted against the time (in min)).

An aqueous solution prepared from a mixture of:
- 10 µl of phosphate-buffered saline,
- 100 µl of reductant (conc: 10 mg/ml),
- 10 µl of a diethanolamine solution and
- 10 µl of acridine salt (conc: $10^{-4}$ M/l).

To this aqueous solution, 880 µl of an alcohol was added and chemiluminescence was recorded for over a 50 min period.

EXAMPLE II: Detection of minute quantities of acridine compounds:

Under alkaline conditions and in the presence of an excess of alcohol, very small quantities of acridine ester can be detected. This is illustrated in Figure 2, in which the intensity of the signal light emission expressed as a logarithm of the relative light intensity units is plotted against the negative logarithm of the concentration of lucigenin.

Serial dilutions of lucigenin were prepared in distilled water. From each dilution, 10 µl was pipetted into appropriate vials. Subsequently 10 µl of a solution of 0.01 M NaCN in distilled water was added. The reaction was started by the addition of 980 µl of an alcohol. As shown, lucigenin concentrations of $10^{-17}$ M result in chemiluminescence counts that are well above the background level when measured in "in coincidence" mode, (i.e. 12 nanoseconds detection period with two photomultipliers).

EXAMPLE III: Sensitive pH measurements in small reaction volumes:

According to the present invention, highly sensitive pH measurements can be obtained relatively simply using small sample volumes.

Solutions of $10^{-3}$ M lucigenin were prepared in buffered solution at a pH ranging from 3.01 to 4.19 of the indicated pH. To 100 µl of each lucigenin solution, 900 µl of n-propyl alcohol was added and the chemiluminescence response was measured and expressed as counts per minute in the "in coincidence

mode". The results of Figure 3, in which the intensity of the light emission is plotted against pH, indicates that under these conditions a pH interval of 1 pH unit is able to generate a span of almost 4 x $10^5$ cpm. Figure 4 shows analogous results in the higher pH range of 8.39 to 9.29.

EXAMPLE IV: Detection of minute quantities of antigens using urease-coupled antibodies:

Urease is an enzyme that liberates ammonia from solutions of ureum. The resulting increase of the pH of a buffered solution can be detected by the chemiluminescence reaction of the present invention. To illustrate this, different dilutions of 0.5 U urease (5 U/mg) were dissolved in 10 ml of a phosphate-buffered solution of pH 7.4, containing $10^{-3}$M lucigenin and 0.1% (v/w) hydroquinone. To 600 µl of each dilution, 10 µl of ureum (0.5 mg/ml in distilled water) were added and the mixture was incubated for 30 min, at 37° C. Chemiluminescence was induced by the addition of 400 µl of ethylalcohol. As shown in Figure 5 (in which the intensity of the light signal - expressed in c.p.m. in coincidence mode - is plotted against the dilution of urease expressed in units/3), less than 0.1 µU of urease can easily be detected within a 30 min reaction time.

This reaction can be used to detect any antigen provided that suitable monoclonal or polyclonal antibodies are available that recognize this antigen. Either these antigen-detecting antibodies can be conjugated with urease using standard coupling techniques or these antibodies can be detected by secondary species-specific antibodies conjugated to urease according to standard techniques. Such labeled antibodies are commercially available. Quantification of an antigen is illustrated using human tumor necrosis factor (HuTNF) as the antigen in an enzyme-linked immunosorbent assay. To microwells coated with rabbit anti-HuTNF, 200 µl of serial dilutions of HuTNF were added (in the range of 2.5 µg/ml - 250 femtograms/ml), prepared in a solution of phosphate-buffered saline, 0.1% bovine serum albumin (BSA) and 0.05% Tween 20. After incubation for 2 h at 37° C, microwell plates were rinsed and 200 µl of a solution of a mouse monoclonal anti-HuTNF tested so as to give optimal binding was added to each well. After incubation for 2 h at 37° C, plates were again rinsed and subsequently incubated with 200 µl of urease-conjugated goat anti-mouse IgG (40 ng). Incubation was continued for another hour at 37° C. After final rinsing each well was filled with 200 µl of urease substrate solution (Sera Labs). After incubation for 1 hour at 37° C, 100 µl of the reaction mixture was pipetted into luminescence vials (LKB). To each vial 50 µl of $10^{-3}$ M lucigenin pH 5.2 was added and luminescence was initiated by addition of 850 µl of ethylalcohol. Figure 6 (on which the intensity of the light signal - expressed in c.p.m. in coincidence mode - is plotted against the concentration of TNF expressed in femtograms/ml, pg/ml, and ng/ml) shows that 50 femtograms/ml of TNF can be measured.

EXAMPLE V: Measurement of alkaline phosphatase activity:

At the optimum pH of alkaline phosphatase (i.e. pH 9.8), enzymatic activity can be measured using buffered solutions of lucigenin and mannose in the presence of an alcohol due to the release of phosphate. If the alkaline phosphatase enzyme is coupled to species-specific antibodies, the reaction can be used to detect and quantify any antigen provided that antigen-specific monoclonal or polyclonal antibodies are available that can be coupled with the enzyme or can react with the enzyme-coupled species-specific antibodies.

This is exemplified by making serial dilutions of equimolar amounts of p-nitrophenol and p-nitrophenolphosphate in diethanolamine buffer at pH 9.8 from which 10 µl can be added to a mixture containing 100 µl of a mannose solution (10 mg/ml in distilled water) and 10 µl of $10^{-4}$ M lucigenin dissolved in distilled water. Chemiluminescence is induced by the addition of 880 µl of ethanol. As can be seen in Figure 7 (on which the absorbance at 405 nm is plotted against dilution of p-nitrophenol in diethanolamine buffer), the extent of the enzymatic reaction can also be followed using the optical density measurements at 405 nm as a consequence of the liberation of p-nitrophenol (colorimetric reaction), but the counting of the luminescence in c.p.m increases the sensitivity of detection considerably (Figure 8, on which the intensity of the light signal - expressed in log of c.p.m. in coincidence - is plotted against the negative logarithm of the concentration of p-nitrophenol).

EXAMPLE VI: Detection and quantification of amino acids:

According to the present invention, chemiluminescence can be used to detect pH changes. If a pH-increasing compound is generated as a consequence of a stoichiometric reaction, any such reaction can be quantified provided that this reaction does not quench the chemiluminescence. A typical example of this principle can be found in the quantification of amino acids using the ninhydrin reaction in which the reaction of ninhydrin with an amino acid results in the liberation of $NH_3$.

In Figure 9 (on which the intensity of the light signal - expressed as the log of c.p.m. in the coincidence mode - is plotted against the negative logarithm of the concentration (M/l) of glycine), results are shown of the chemiluminescence produced by mixing 100 µl of serial dilutions of glycine in saline with 100 µl of $10^{-3}$ M lucigenin, 10 µl of a 0.1% (v/w) hydroquinone solution, 780 µl of ethanol and 10 µl of a 1% (v/w) ninhydrin solution. As shown, a concentration of $10^{-17}$ M glycine still produces a signal that is well above background level.

EXAMPLE VII: DNA hybridization assay:

In accordance with the present invention, chemiluminescence can also be used as a tool for the quantitative and qualitative detection of hybridization using the above-mentioned methods to obtain chemiluminescent signals. This is illustrated in Figure 10. Here varying amounts of Neisseria gonorrhoeae DNA were applied to nitrocellulose paper strips. The strips were dried, baked under vacuum at 80° C for 2 hours, and subjected to hybridization with a highly specific DNA probe prelabeled with digoxigenin (Boehringer, Mannheim) according to the protocol supplied by the manufacturer. After hybridization for 16 hours at 65° C in 3 x SSC (SSC = 0.15 M NaCl, 0.015 M sodium citrate), 0.1% (w/v) N-laurylsarcosine, 0.02% (w/v) sodium dodecyl sulfate and 0.5% blocking reagent, the filters were washed repeatedly with 1.5 x SSC or 3 x SSC and incubated with alkaline phosphatase-labeled anti-digoxigenin antibodies as specified by the supplier. After binding and washing, the enzymatic reaction was allowed to proceed in the presence of 1 mg/ml p-nitrophenylphosphate in diethanolamine buffer, pH 9.8 for 12 hours at room temperature. An aliquot of the incubation mixture of each of the different filters was pipetted into a solution of 100 µl mannose (of concentration of 10 mg/ml) and 10 µl of $10^{-4}$ M lucigenin. After the addition of 880 µl of ethylalcohol chemiluminescence was measured at different time periods. The results of Figure 10 (on which the intensity of the light signal - expressed in log of c.p.m. in coincidence - is plotted as a function of time after the addition of the alcohol for the different concentrations of DNA) indicate that a stable signal is generated and that amounts of 10 femtograms of DNA per filter still can be detected under these circumstances.

EXAMPLE VIII: Detection of acridine compounds contained within liposomes:

Lucigenin containing liposomes were prepared by dissolving 4 mg dipalmitoylphosphatidylcholine (DPPC) in chloroform ($CHCl_3$). After evaporation of the $CHCl_3$ (1 hour in a $N_2$ atmosphere), 600 µl of a lucigenin solution (13 mg/ml in phosphate-buffered saline, pH 7.4) was added. The solution was then sonicated for 4 x 7 min, 32 W (Branson sonifier with stepped microtip) at a temperature above the transition temperature of the DPPC.

Next, enough sonicated solution, containing 0,8 mg of DPPC was separated on a Sepharose 6B using a 25 x 1 cm column. During the run, two fractions could be separated, one of which contained the lucigenin encapsulated in the liposomes. Furthermore it could be established that this fraction did not contain free lucigenin.

Next, a serial dilution (1/10 - 1/$10^7$) of the liposome-containing fraction was made in physiological saline, pH 9.

After the addition of 10 µl Triton X-100 (0.01%) to 100 µl liposome suspension, 900 µl ethanol was added and the chemiluminescence (c.p.m. in coincidence mode) was recorded.

Maximum peak heights (expressed as log c.p.m. in coincidence mode) are represented in Table 1 below:

TABLE 1

| Liposome fraction dilution | log c.p.m. In coincidence mode |
|---|---|
| 0 | 2.40 |
| 1/10 | 6.46 |
| $1/10^2$ | 6.25 |
| $1/10^3$ | 5.44 |
| $1/10^4$ | 3.94 |
| $1/10^5$ | 3.31 |
| $1/10^6$ | 3.24 |
| $1/10^7$ | 2.90 |

EXAMPLE IX: Detection and quantification of reducing compounds:

According to the present invention chemiluminescence may be used to detect reducing compounds. In Figure 11 the concentration of ascorbate is plotted against the intensity of the light signal expressed in c.p.m., obtained by mixing 150 µl 0.1 mM lucigenin, dissolved in distilled water, with 100 µl ascorbate, dissolved according to the specific concentrations in phosphate-buffer saline, and 750 µl ethanol. As shown, 10 femtograms of ascorbate in the reaction mixture can easily be detected.

EXAMPLE X: Detection of alkaline phosphatase with lucigenin-BCIP chemiluminescence:

Calf intestinal alkaline phosphatase (CIP) (Boehringer) was diluted (1/5000) in 2% diethanolamine buffer (DEA), pH 9.41 containing 0.01% albumin. Starting from this enzyme preparation, further dilutions in steps of 1/10 were made in the same buffer (1/5000 - 1/5 x $10^{14}$).

Lucigenin was first dissolved at a concentration of $10^{-3}$ M in 2% diethanolamine buffer pH 9.41 and further diluted to $10^{-4}$ M in the same buffer.

50 mg bromo-chloro-indolylphosphate (sodium salt) (Boehringer) hereafter designated by BCIP was dissolved in 1 ml 2% DEA buffer, pH 9.41. 350 µl of this solution was added to the lucigenin ($10^{-4}$ M) containing DEA-buffer.

A second dilution series of CIP was prepared in 2% DEA buffer pH 9.41 containing 7% of polyvinylalcohol (PVA) (MW 80,000) (Aldrich) and corrected to pH 9.41 by the addition of NaOH. Finally, albumin was added at a final concentration of 0.01%.

From each of the enzyme dilutions, 250 µl was pipetted into luminescence vials (with a total working volume of 1 ml (LKB 1251) and placed in a scintillation counter (Beckman LS7500)).

Chemiluminescence was initiated by the addition to the samples of 250 µl of the lucigenin-BCIP solution in DEA, at time intervals of 12 seconds. Luminescence was then recorded for a period of 30 min in the "in coincidence mode".

Counts obtained are shown in Table 2.

Here the final concentration of the enzyme in the luminescence samples is given together with the log cpm obtained in DEA and in DEA + PVA. These cpm are compared with the optical density (OD) of the BCIP reaction in DEA-buffer after 30 min when read on a spectrophotometer (Pye Unicam) with automatic background subtraction. The final concentration of the enzyme was the same as for the chemiluminescence assays.

As shown in Table 2, a thousand to ten thousandfold increase in alkaline phosphatase detectability is obtained with the PVA-supplemented chemiluminescence systems.

TABLE 2

| CIP | | log cpm without PVA | log cpm with PVA | OD 620 nm of BCIP |
|---|---|---|---|---|
| Blank | | 1.83 | 1.60 | 0.000 |
| 0.0003 | attomole | 1.80 | 1.80 | 0.000 |
| 0.003 | attomole | 1.50 | 1.85 | 0.000 |
| 0.03 | attomole | 1.71 | 2.38 | 0.000 |
| 0.35 | attomole | 1.64 | 3.20 | 0.000 |
| 3.5 | attomole | 1.55 | 4.55 | 0.000 |
| 35 | attomole | 1.68 | 5.48 | 0.000 |
| 350 | attomole | 2.35 | 6 | 0.003 |
| 3,500 | attomole | 3.75 | 6 | 0.008 |
| 35,000 | attomole | 6 | 6 | 0.080 |
| 350,000 | attomole | 6 | 6 | 0.380 |
| 3,500,000 | attomole | 6 | 6 | 0.675 |

EXAMPLE XI: Comparative examples:

Chemiluminescence obtained with the compositions of the invention and chemiluminescence obtained with prior art compositions containing no alcohol, have been compared.

1) Chemiluminescence obtained with compositions derived from the compositions described in Photochemistry and Photobiology, 1975, Vol. 22, p. 203-211, Totter J.R.

The compositions used are the two following ones (a) and b) respectively):

a) 200 µl distilled water,
100 µl NaOH (molarity as mentioned in Table 3 hereafter),
100 µl lucigenin (2 x $10^{-4}$ M).

b) 100 µl distilled water,
100 µl fructose ($10^{-2}$ M),
100 µl NaOH (molarity as mentioned in Table 3 hereafter),
100 µl lucigenin (2 x $10^{-4}$ M).

The chemiluminescence results (expressed in log of cpm) are shown in Table 3 in which:

- the first column corresponds to the molarity of NaOH,
- the second column shows the log of cpm obtained with compositions (a) hereabove defined and
- the third column shows the log of cpm obtained with compositions (b) defined hereabove.

TABLE 3

| No. | NaOH added (M) | log cpm (without fructose) | log cpm (with fructose) |
|---|---|---|---|
| 1 | 5 x 10-5 | 1.30 | 1.60 |
| 2 | 1 x 10-4 | 1.54 | 1.69 |
| 3 | 5 x 10-4 | 1.65 | 2.24 |
| 4 | 1 x 10-3 | 2.04 | 3.29 |
| 5 | 5 x 10-3 | 2.85 | 5.39 |
| 6 | 1 x 10-2 | 4.10 | 6.77 |
| 7 | 5 x 10-2 | 5.25 | 6.77 |
| 8 | 1 x 10-1 | 6.09 | 6.77 |

2) Chemiluminescence obtained with the two following compositions of the invention, respectively (c) and (d):

c) 100 µl distilled water,
100 µl NaOH (molarity as mentioned in Table 4 hereafter),
100 µl ethanol (EtOH),
100 µl lucigenin (2 x $10^{-4}$ M).
d) 100 µl fructose ($10^{-2}$ M),
100 µl NaOH (molarity as mentioned in Table 4 hereafter),
100 µl ethanol (EtOH),
100 µl lucigenin (2 x $10^{-4}$ M).

The chemiluminescence results (expressed in log of cpm) are summarized in Table 4 in which:
- the first column corresponds to the molarity of NaOH,
- the second column shows the log of cpm obtained with compositions (c) hereabove defined and
- the third column gathers the log of cpm obtained with compositions (d) defined hereabove.

TABLE 4

| No. | NaOH added (M) | log cpm (without fructose) | log cpm (with fructose) |
|---|---|---|---|
| 1 | 5 x 10-5 | 1.30 | 1.84 |
| 2 | 1 x 10-4 | 1.30 | 3.01 |
| 3 | 5 x 10-4 | 3.26 | 5.05 |
| 4 | 1 x 10-3 | 4.66 | 6.77 |
| 5 | 5 x 10-3 | 6.77 | 6.77 |
| 6 | 1 x 10-2 | 6.77 | 6.77 |
| 7 | 5 x 10-2 | 6.77 | 6.77 |
| 8 | 1 x 10-1 | 6.77 | 6.77 |

Conclusion 1:

As shown in Tables 3 and 4, the system clearly demonstrates that the use of fructose results in substantially higher quantum efficiency of chemiluminescence compared with that in the absence of fructose. In effect, the compositions of the invention are of substantially greater quantum efficiency than the

compositions described by Totter J.R. (Photochemistry and Photobiology, 1975, Vol. 22, p. 203-211).

3) Chemiluminescence obtained with prior art compositions: 1, 2, 3, 4, 5, 6, 7, 8, 9 and 10, hereafter defined and compositions of the invention: 1', 2', 3', 4', 5', 6', 7', 8', 9', and 10', hereafter defined.

Prior art compositions:

1: 100 µl NaOH $10^{-1}$ M + 100 µl fructose $10^{-2}$ M + 100 µl distilled water + 100 µl lucigenin $10^{-3}$ M;
2: same as 1, in which there is 200 µl instead of 100 µl distilled water,
3: same as 1, in which there is 300 µl instead of 100 µl distilled water,
4: same as 1, in which there is 400 µl instead of 100 µl distilled water,
5: same as 1, in which there is 500 µl instead of 100 µl distilled water,
6: same as 1, in which there is 600 µl instead of 100 µl distilled water,
7: same as 1, in which there is 700 µl instead of 100 µl distilled water,
8: same as 1, in which there is 800 µl instead of 100 µl distilled water,
9: same as 1, in which there is 900 µl instead of 100 µl distilled water,
10: same as 1, in which there is 1000 µl instead of 100 µl distilled water.

Compositions of the invention:

1': 100 µl NaOH $10^{-1}$ M + 100 µl fructose $10^{-2}$ M + 100 µl EtOH + 100 µl lucigenin $10^{-3}$ M,
2': same as 1', in which there is 200 µl instead of 100 µl distilled water,
3': same as 1', in which there is 300 µl instead of 100 µl distilled water,
4': same as 1', in which there is 400 µl instead of 100 µl distilled water,
5': same as 1', in which there is 500 µl instead of 100 µl distilled water,
6': same as 1', in which there is 600 µl instead of 100 µl distilled water,
7': same as 1', in which there is 700 µl instead of 100 µl distilled water,
8': same as 1', in which there is 800 µl instead of 100 µl distilled water,
9': same as 1', in which there is 900 µl instead of 100 µl distilled water,
10': same as 1', in which there is 1000 µl instead of 100 µl distilled water,

The results are summarized in Table 5, in which:

- the first column refers to the compositions as defined above,
- the second column shows the cpm obtained at the start of the chemiluminescence reaction,
- the third column shows the cpm obtained 10 hours after the beginning of the reaction,
- the fourth column shows the cpm obtained 24 hours after the beginning of the reaction,
- the fifth column shows the cpm obtained 96 hours after the beginning of the reaction.

TABLE 5

| No. | cpm start | after 10 hours | after 24 hours | after 96 hours |
|---|---|---|---|---|
| 1 | 6,000,000 | 114,829 | 40,045 | 1,937 |
| 1′ | 6,000,000 | 1,067,937 | 863,062 | 77,962 |
| 2 | 6,000,000 | 84,639 | 44,093 | 1,871 |
| 2′ | 6,000,000 | 6,000,000 | 6,000,000 | 6,000,000 |
| 3 | 6,000,000 | 95,548 | 50,379 | 3,346 |
| 3′ | 6,000,000 | 6,000,000 | 6,000,000 | 6,000,000 |
| 4 | 6,000,000 | 102,488 | 30,446 | 4,527 |
| 4′ | 6,000,000 | 6,000,000 | 6,000,000 | 6,000,000 |
| 5 | 6,000,000 | 902,453 | 38,983 | 2,878 |
| 5′ | 6,000,000 | 6,000,000 | 6,000,000 | 6,000,000 |
| 6 | 6,000,000 | 6,000,000 | 6,000,000 | 4,448 |
| 6′ | 6,000,000 | 6,000,000 | 6,000,000 | 6,000,000 |
| 7 | 6,000,000 | 6,000,000 | 6,000,000 | 3,183 |
| 7′ | 6,000,000 | 6,000,000 | 6,000,000 | 6,000,000 |
| 8 | 6,000,000 | 6,000,000 | 6,000,000 | 1,419,279 |
| 8′ | 6,000,000 | 6,000,000 | 6,000,000 | 6,000,000 |
| 9 | 6,000,000 | 6,000,000 | 6,000,000 | 2,894,977 |
| 9′ | 6,000,000 | 6,000,000 | 6,000,000 | 6,000,000 |
| 10 | 6,000,000 | 6,000,000 | 6,000,000 | 6,000,000 |
| 10′ | 6,000,000 | 6,000,000 | 6,000,000 | 6,000,000 |

Conclusion 2:

The results of Table 5 Clearly demonstrates the efficiency of the compositions of the invention, which comprise the use of a water-miscible alcohol. Substantial improvement in quantum efficiency and an extended duration of chemiluminescence with the compositions of the invention are demonstrated which have not been previously observed.

4) Chemiluminescence obtained with the prior art compositions 1, 2, 3, 4, 5, 6, 7, 8, 9, 10, 11, 12, 13, 14, 15 and 16 defined hereafter and the compositions of the invention 1′, 2′, 3′, 4′, 5′, 6′, 7′, 8′, 9′, 10′, 11′, 12′, 13′, 14′, 15′ and 16′ defined hereafter.

Prior art compositions: 1, 3, 5, 7, 9, 11, 13 and 15 are the following ones:
- 100 µl distilled water,
- 100 µl NaOH (molarity as mentioned in Table 6),
- 100 µl $H_2O$,
- 100 µl lucigenin ($10^{-4}$ M).

Prior art compositions 2, 4, 6, 8, 10, 12, 14 and 16 correspond respectively to compositions 1, 3, 5, 7, 9, 11, 13 and 15 in which 100 µl distilled water is replaced by 100 µl fructose $10^{-2}$ M.

Compositions of the invention 1′, 3′, 5′, 7′, 9′, 11′, 13′ and 15′ are the following ones:
- 100 µl distilled water,
- 100 µl NaOH (molarity as mentioned in Table 6),
- 100 µl EtOH,
- 100 µl lucigenin ($10^{-4}$ M).

Compositions of the invention 2′, 4′, 6′, 8′, 10′, 12′, 14′ and 16′ correspond respectively to compositions 1′, 3′, 5′, 7′, 9′, 11′, 13′ and 15′ defined above in which 100 µl distilled water is replaced by 100 µl fructose $10^{-2}$ M.

The chemiluminescence results obtained at 60 min after onset of the reaction are expressed in Table 6:

TABLE 6

| Prior art composition | NaOH | cpm | Invention composition | cpm |
|---|---|---|---|---|
| 1 | 5 x 10-5 | 30 | 1' | 35 |
| 2 | 5 x 10-5 | 10 | 2' | 25 |
| 3 | 1 x 10-4 | 20 | 3' | 40 |
| 4 | 1 x 10-4 | 55 | 4' | 50 |
| 5 | 5 x 10-4 | 40 | 5' | 30 |
| 6 | 5 x 10-4 | 20 | 6' | 20 |
| 7 | 1 x 10-3 | 35 | 7' | 35 |
| 8 | 1 x 10-3 | 25 | 8' | 15 |
| 9 | 5 x 10-3 | 35 | 9' | 120 |
| 10 | 5 x 10-3 | 1,325 | 10' | 1,090,960 |
| 11 | 1 x 10-2 | 65 | 11' | 8,825 |
| 12 | 1 x 10-2 | 603,420 | 12' | 6,000,000 |
| 13 | 5 x 10-2 | 4,910 | 13' | 278,035 |
| 14 | 5 x 10-2 | 6,000,000 | 14' | 6,000,000 |
| 15 | 1 x 10-1 | 83,065 | 15' | 6,000,000 |
| 16 | 1 x 10-1 | 6,000,000 | 16' | 6,000,000 |

Conclusion 3:

As previously described in conclusions 1 and 2, both an increase in quantum efficiency and long-lived chemiluminescence over a period of more than 60 min is demonstrated using the compositions of the invention. The effect of base compositions and reductant has a clear enhancing effect on the light emission without the prior use of hydrogen peroxide.

## Claims

1. Homogeneous hydroalcoholic chemiluminescent composition comprising:
- a solution containing an acridine derivative, said acridine derivative being either water-soluble or alcohol-soluble or can be solubilized in the homogeneous hydroalcoholic composition, said solution being preferably aqueous,
- a water-miscible alcohol, which is either water-soluble or forms in water a suspension that is sufficiently stable to permit the chemiluminescent reaction to take place, provided that the alcohol is different from an alcohol derived from an acridine derivative and is different from acridanol and is different from an alcohol derived from an acridanol derivative, which might be formed during the chemiluminescence reaction,
- a reducing agent, which can be either present or absent, depending upon the appropriate conditions of pH.

2. Homogeneous hydroalcoholic chemiluminescent composition, according to Claim 1, wherein the water-miscible alcohol is a primary, secondary, tertiary, aliphatic or a primary, secondary, tertiary, aromatic alcohol or a primary, secondary, tertiary, heterocyclic alcohol excluding sugars and including mercapto alcohols.

3. Homogeneous hydroalcoholic chemiluminescent composition, according to Claim 1 or 2, wherein the alcohol is different from a mercapto alcohol.

4. Chemiluminescent composition according to anyone of Claims 1 to 3 comprising:
- a solution of an acridine derivative, said solution being preferably aqueous,
- a water-miscible alcohol,
- the pH of the solution being such that the alcohol can provoke the chemiluminescent reaction.

5. Chemiluminescent composition according to anyone of Claims 1 to 3 comprising:
- a solution of an acridine derivative, said solution being preferably aqueous,
- a water-miscible alcohol,

- a base such that the pH of the solution is adjusted at a value at which the alcohol can provoke the chemiluminescent reaction.

6. Chemiluminescent composition according to anyone of Claims 1 to 3 comprising:
- a solution of an acridine derivative and of a reducing agent, said solution being preferably aqueous,
- a water-soluble alcohol,
- possibly a base.

7. Chemiluminescent composition according to anyone of Claims 1 to 6 comprising an aqueous buffer.

8. Chemiluminescent composition according to anyone of Claims 1 to 7, comprising an enzyme, such as urease, alkaline phosphatase or peroxydase.

9. Chemiluminescent composition according to anyone of Claims 1 to 8, wherein the acridine derivative has the following formula:

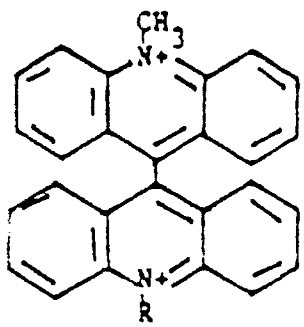

$2X^-$ or $X^{2-}$

wherein $2X^-$ or $X^{2-}$ represents a negatively charged counterion such as a nitrate, sulfate, phosphate, chlorate,
R represents an aliphatic chain of 1 to 6 carbon atoms, particularly 1 to 4 carbon atoms, and more particularly $CH_3$, with at least one of the phenyl rings being optionally substituted provided that the acridine derivative is either water-soluble or alcohol-soluble or can be solubilized in the homogeneous hydroalcoholic composition, preferably water-soluble,
said acridine derivative being preferably lucigenin,
or

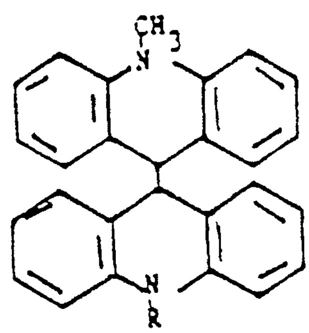

wherein R has the above-mentioned meaning,
with at least one of the phenyl rings being optionally substituted provided that the acridine derivative is water-miscible, either water-soluble or alcohol-soluble or can be solubilized in the homogeneous hydroalcoholic composition, preferably water-soluble.

10. Chemiluminescent composition according to anyone of Claims 1 to 8, wherein the acridine derivative has the following formula:

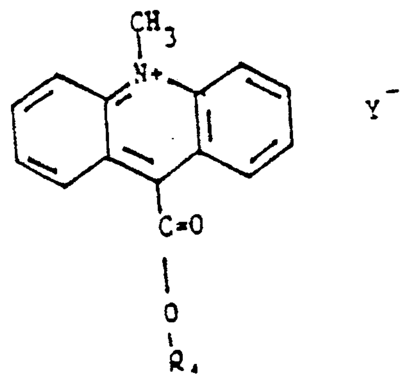

wherein Y represents a negatively charged counterion such as $FSO_3^-$, $NO_3^-$,
$R_1$ represents

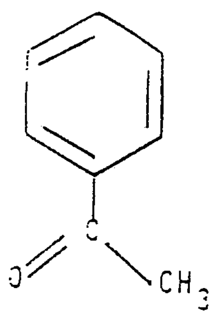 , 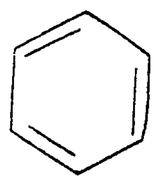 , 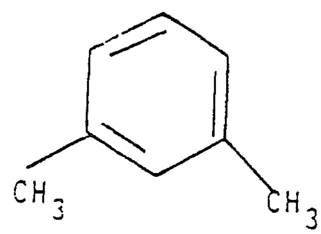 .

at least one of the phenyl rings being liable to be substituted provided that the acridine derivative is either water-soluble or alcohol-soluble or can be solubilized in the homogeneous hydroalcoholic composition, preferably water-soluble,
or

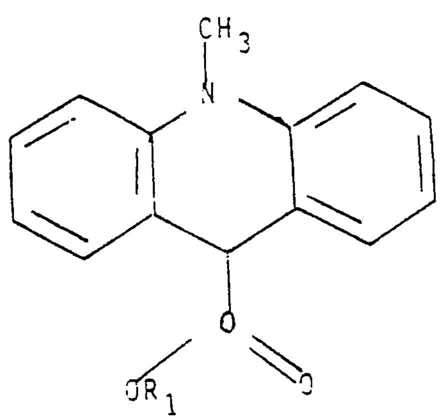

wherein $R_1$ has the above-mentioned meaning,
at least one of the phenyl rings being liable to be substituted provided that the acridine derivative is either water-soluble or alcohol-soluble or can be solubilized in the homogeneous hydroalcoholic composition, preferably water-soluble.

11. Chemiluminescent composition according to anyone of Claims 1 to 10, wherein the alcohol is a water-soluble primary, secondary or tertiary alcohol of 1 to 6 carbon atoms, particularly 1 to 4 carbon atoms, and more particularly methanol, ethanol, n-propanol, isopropanol, or butanol.

12. Chemiluminescent composition according to anyone of Claims 1, 2, 3, 5 to 11, wherein the reducing agent is generated in situ .

13. Chemiluminescent composition according to anyone of Claims 1, 2, 3, 5 to 12, wherein the reducing agent has an oxido-reduction potential of at least 0.3V and does not interfere with the molecular structure of the acridine derivative, especially with the intermediate compound(s) responsible for the chemiluminescent reaction, with the said reducing agent being, for instance, ascorbic acid, mannose, glucose, NADH or NADPH, or hydroquinone.

14. Chemiluminescent composition according to anyone of Claims 1 to 13, wherein the buffer is a phosphate buffer, Tris buffer, acetate buffer, diethanolamine buffer, barbital buffer, citrate buffer.

15. Chemiluminescent composition according to anyone of Claims 1 to 14, wherein the

- acridine derivative: from about 0.01% to about 99.99% (in volume), particularly from about 10% to about 99% of a concentration range from about $10^{-4}$ to $10^{-10}$ M/l, especially from about a range to $10^{-4}$ to about $10^{-6}$ M/l
- alcohol: volume from about 0.5% to about 99.9%, particularly from about 10% to about 90%, all these percentages are expressed in volumes with respect to the total volume of the composition.
- the pH is from about 4 to about 8, particularly from about 6 to about 7.6.

16. Luminescent or luminometric assay wherein the chemiluminescent reaction is based on the chemiluminescent composition according to anyone of Claims 1 to 15.

17. Process for determining the concentration of an acridine derivative in an aqueous solution according to the assay of Claim 16, wherein the concentration of the reducing agent is constant, the concentration of the alcohol is constant and the pH is constant, the rate of the light produced in the chemiluminescent reaction being a function of the acridine derivative concentration.

18. Process for determining the concentration of a reducing agent in an aqueous solution according to the assay of Claim 16, wherein the concentration of the acridine derivative, the concentration of the alcohol are constant and the pH is constant, the rate of the light produced in the chemiluminescent reaction being a function of the reducing agent concentration.

19. Process for determining the pH of aqueous solutions, particularly of minute amounts of solute, for example of less than 10 µl, particularly from about 0.1 µl to about 1 µl, according to the assay of Claim 16, wherein the concentration of the acridine derivative is constant, the concentration of the alcohol is constant and the concentration of the reducing agent is constant, the rate of the light produced in the chemiluminescent reaction being a function of the pH.

20. Process according to Claim 19, wherein the pH is generated in situ either in a stoichiometric way or in a time-dependent way.

21. Process according to Claim 16, wherein the concentration of the acridine compounds and the concentration of the water-soluble compounds are constant and a constant amount of reducing agent is generated upon reaching or exceeding a predetermined pH either in a stoichiometric or time-dependent fashion.

22. Process for quantifying pH-increasing substance, according to Claim 16, comprising the introduction of a pH-increasing substance, such as basic or polybasic ions or enzymes generating such ions such as urease generating $NH_4^+$ into a solution of acridine derivative and of alcohol, the rate of light produced being a function of the pH-increasing substance.

23. Process for quantifying antigens or antibodies according to Claim 16, comprising the introduction of a solution containing the antigen to be quantified bound to a pH-increasing substance, or an antibody to be quantified bound to a pH-increasing substance, into a solution of acridine derivative and of alcohol advantageously in the presence of a reducing agent, with the rate of the light produced being a function of the pH-increasing substance from which the quantification of antigen or antibody can be deduced by comparison with calibration curves.

24. Process for measuring alkaline phosphate activity on a substrate containing phosphate according to Claim 16, wherein the chemiluminescent composition comprises buffered solutions of lucigenin and mannose in the presence of an alcohol, wherein the rate of light produced in the chemiluminescent reaction is a function of phosphate groups released by the action of alkaline phosphatase on the substrate.

25. Process for determining antigen or antibody in an assay according to Claim 16, wherein the antigen or antibody which is to be determined is bound to alkaline phosphatase, which is reacted on a substrate containing phosphate groups, such as ATP, 1,2-glycerophosphate, p-nitrophenylphosphate, indolylphosphate, wherein the rate of light produced in the chemiluminescent reaction is a function of phosphate groups released by the action of alkaline phosphatase on the substrate or wherein the antigen or antibody which is to be determined is bound to a substrate containing phosphate group(s) such as ATP, 1,2-glycerophosphate, p-nitrophenylphosphate, β-glycerophosphate, phosphocreatine, which is reacted on alkaline phosphatase, with the rate of light produced in the chemiluminescent reaction being a function of phosphate groups released by the action of alkaline phosphatase on the substrate.

26. Process for determining quantitative and qualitative detection of hybridization reaction of a probe with a nucleic acid to be detected involving the assay of Claim 16, wherein the probe is prelabeled with a label, an alkaline phosphatase-labeled antibody (monoclonal or polyclonal) against the said label being used for the enzymatic reaction to take place between the alkaline phosphatase and a substrate containing phosphate group(s), the rate of the light produced in the chemiluminescent reaction being a function of phosphate groups released by the alkaline phosphatase.

27. Process for detecting and quantifying amino acids according to the assay of Claim 16, comprising addition of ninhydrin into a chemiluminescent composition of the invention containing the amino acid to be detected and quantified, determining the amount of the ninhydrin which has reacted with the amino acid to be detected and quantified, and consequently determining the amount of the amino acid to be detected and quantified by comparison with calibration curves.

28. Kit for the detection and quantification of any antigen (or antibody) present in a test medium such as body fluids and cell supernatants and which comprises:

- means for the fixation of an antibody (or antigen) liable to form an immunological complex with the antigen (or antibody) to be determined,
- a monoclonal or polyclonal antibody forming a complex with a pH-increasing substance or a pH-increasing enzyme such as urease or a phosphate-generating enzyme such alkaline phosphatase, with the said antibody being able to form a specific bond with the antigen (or antibody) to be determined,
- a chemiluminescent composition according to anyone of Claims 1 to 15.

29. Kit for the detection and quantification of any antigen (or antibody) present in a test medium such as body fluids and cell supernatants and which comprises:

- a solid support preferably microwells, plastic luminometer vials or protein binding membranes, to which proteins (i.e. antibody when the substance to be determined is an antigen, or antigen when the substance to be determined is an antibody) have been bound, covalently or non-covalently, with the said proteins being able to form an immunological complex with the antigen (or antibody) to be determined in the test medium,
- a monoclonal or polyclonal antibody forming a complex with a pH-increasing substance or a pH-increasing enzyme such as urease or a phosphate-generating enzyme such as alkaline phosphatase and liable to form a specific bond with the antigen (or antibody) to be determined,
- a chemiluminescent composition according to anyone of Claims 1 to 15.

30. Kit for detecting and quantifying a particular target nucleic acid sequence in a test medium, comprising in one or more containers:

- a first single-stranded nucleotide probe capable of hybridizing with a nucleotide sequence present in the target nucleic acid sequence to be detected, with the said first probe being fixed on fixation means,
- a second chemically or enzymatically labeled nucleotide probe capable of hybridizing with a nucleotide sequence present in the target nucleic acid sequence, with the said second probe not being able to hybridize with the first probe fixed on fixation means,
- means for detecting the label of the second probe, with the said means being conjugated to urease or alkaline phosphatase or lucigenin-loaded liposomes,
- a chemiluminescent composition according to anyone of Claims 1 to 15.

31. Kit for detecting and quantifying a particular target nucleic acid sequences in a test medium, comprising in one or more containers:

- a first polynucleotide probe comprising a single-stranded nucleotide sequence capable of hybridizing with the target nucleic acid sequence to be detected, with the said first probe being fixed to a solid support preferably in the form of strips, microtiter plates, or plastic luminometer vials
- a second chemically or enzymatically labeled nucleotide probe (for instance labeled with biotin, digoxigenin) capable of hybridizing with a nucleotide sequence present in the target nucleic acid sequence, with the said second probe not being able to hybridize with the first probe fixed to the solid support,
- a protein or any other substance which can be attachable to the said label, with the said protein or any other substance being, for example, avidin or anti-digoxigenin antibodies and being conjugated to urease or alkaline phosphatase or lucigenin-loaded liposomes,
- an appropriate chemiluminescent composition of anyone of Claims 1 to 15.

FIGURE 1
LONGEVITY OF THE CHEMILUMINESCENCE SIGNAL

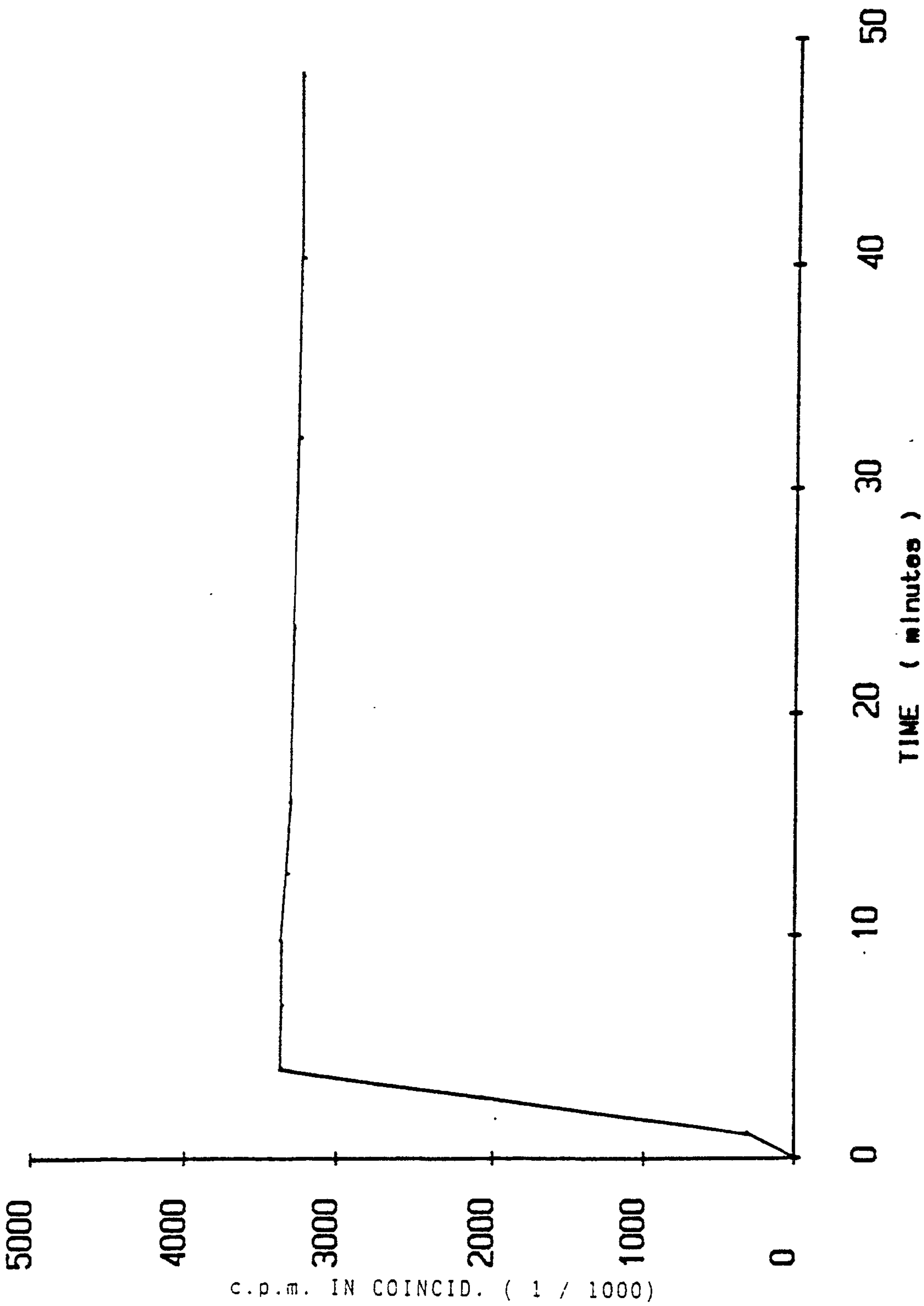

FIGURE 2

DETECTION OF ACRIDINIUM COMPOUND.

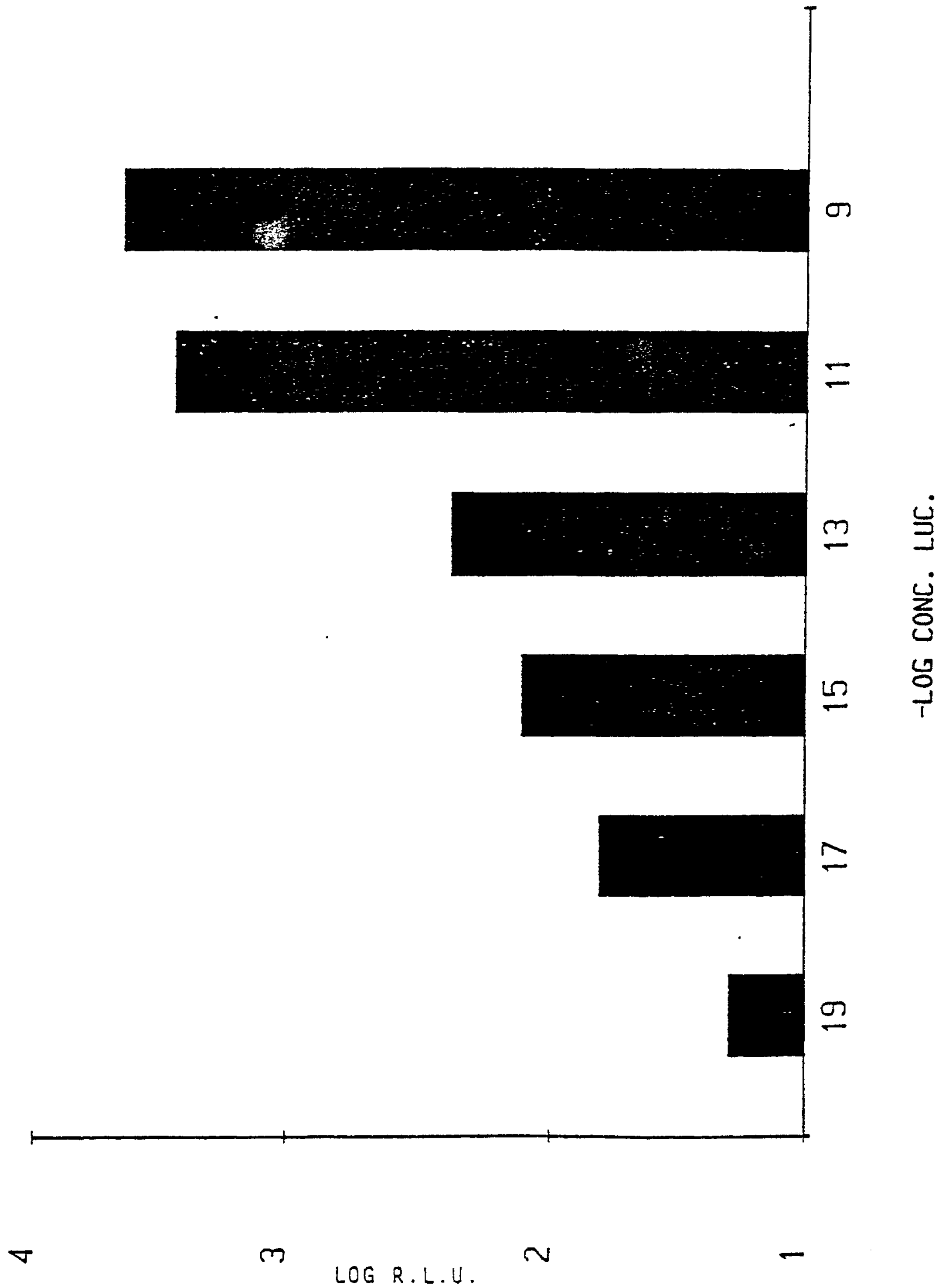

FIGURE 3

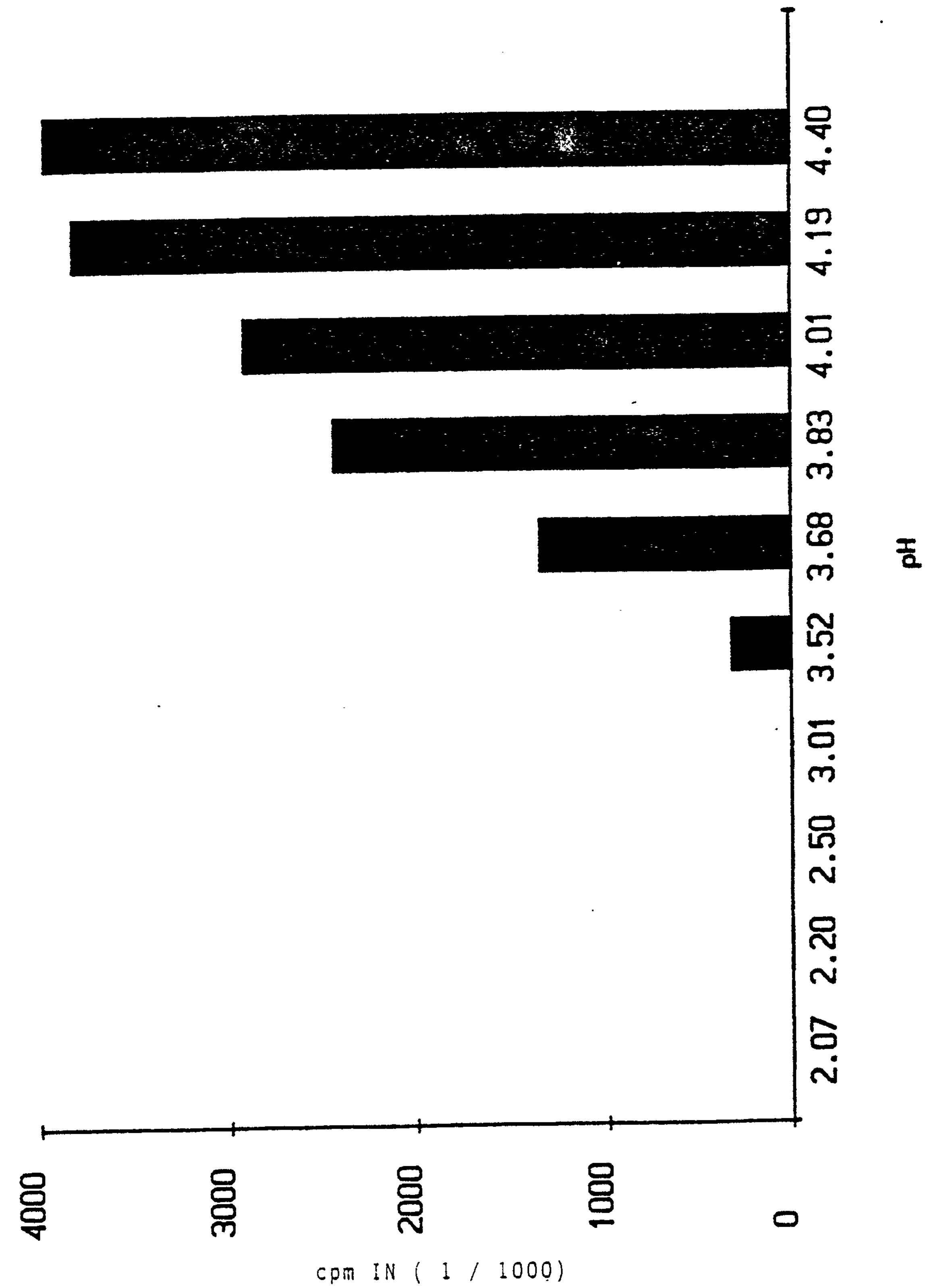
4000
3000
2000
1000
0
cpm IN ( 1 / 1000)
2.07
2.20
2.50
3.01
3.52
3.68
3.83
4.01
4.19
4.40
pH

FIGURE 4

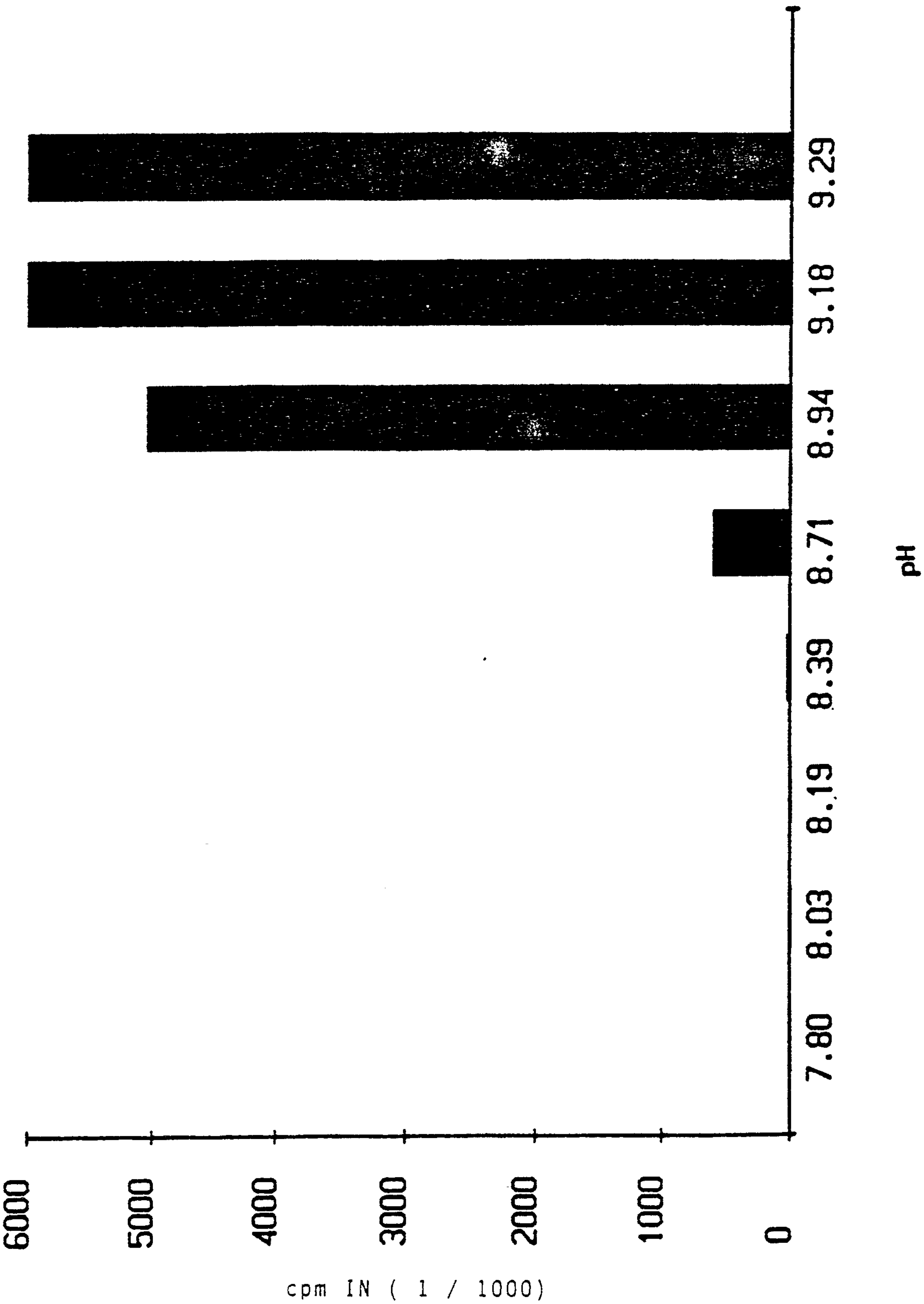
6000
5000
4000
3000
2000
1000
0
cpm IN ( 1 / 1000)
7.80
8.03
8.19
8.39
8.71
8.94
9.18
9.29
pH

FIGURE 5

DETECTION OF UREASE.

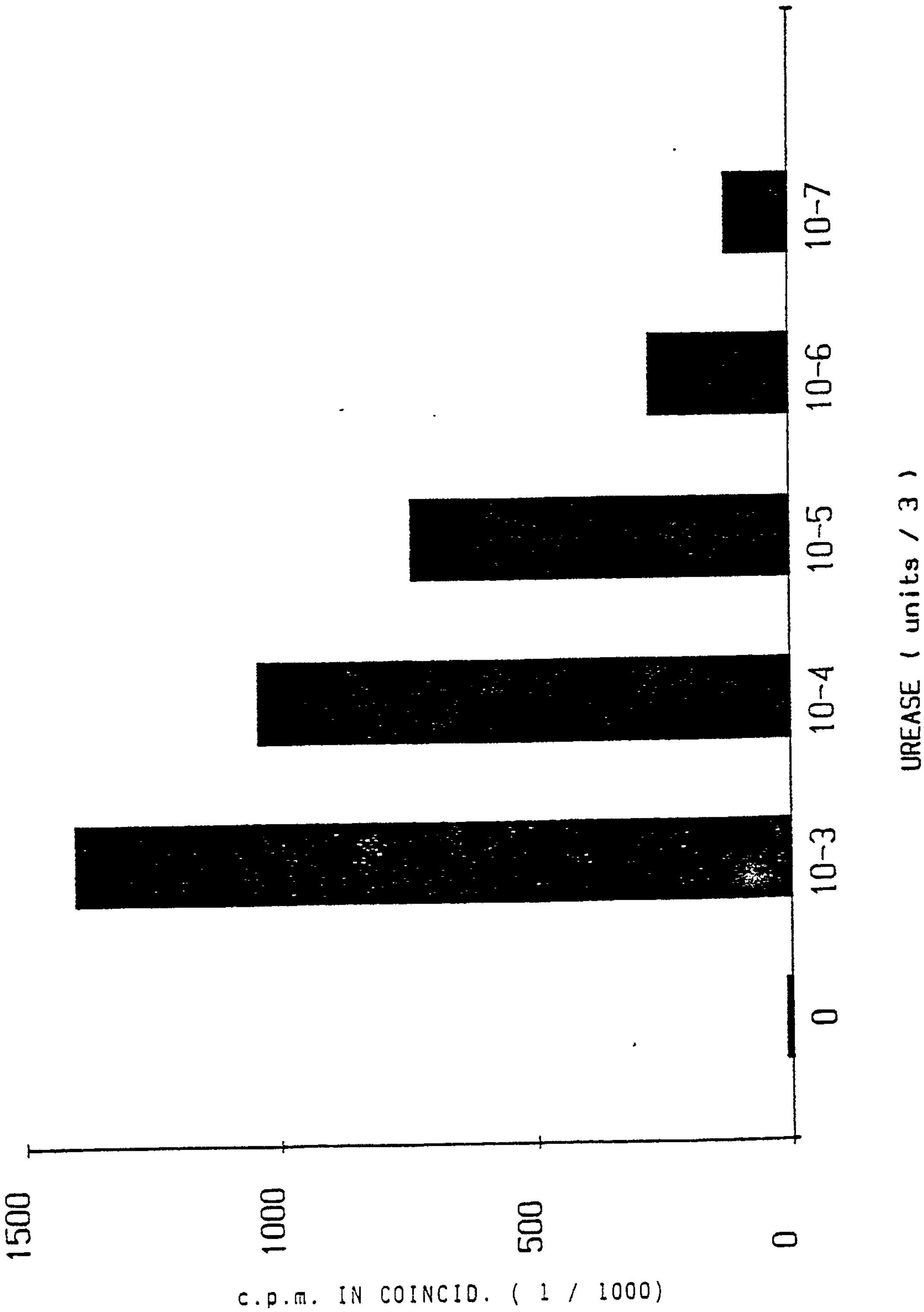

Figure 6

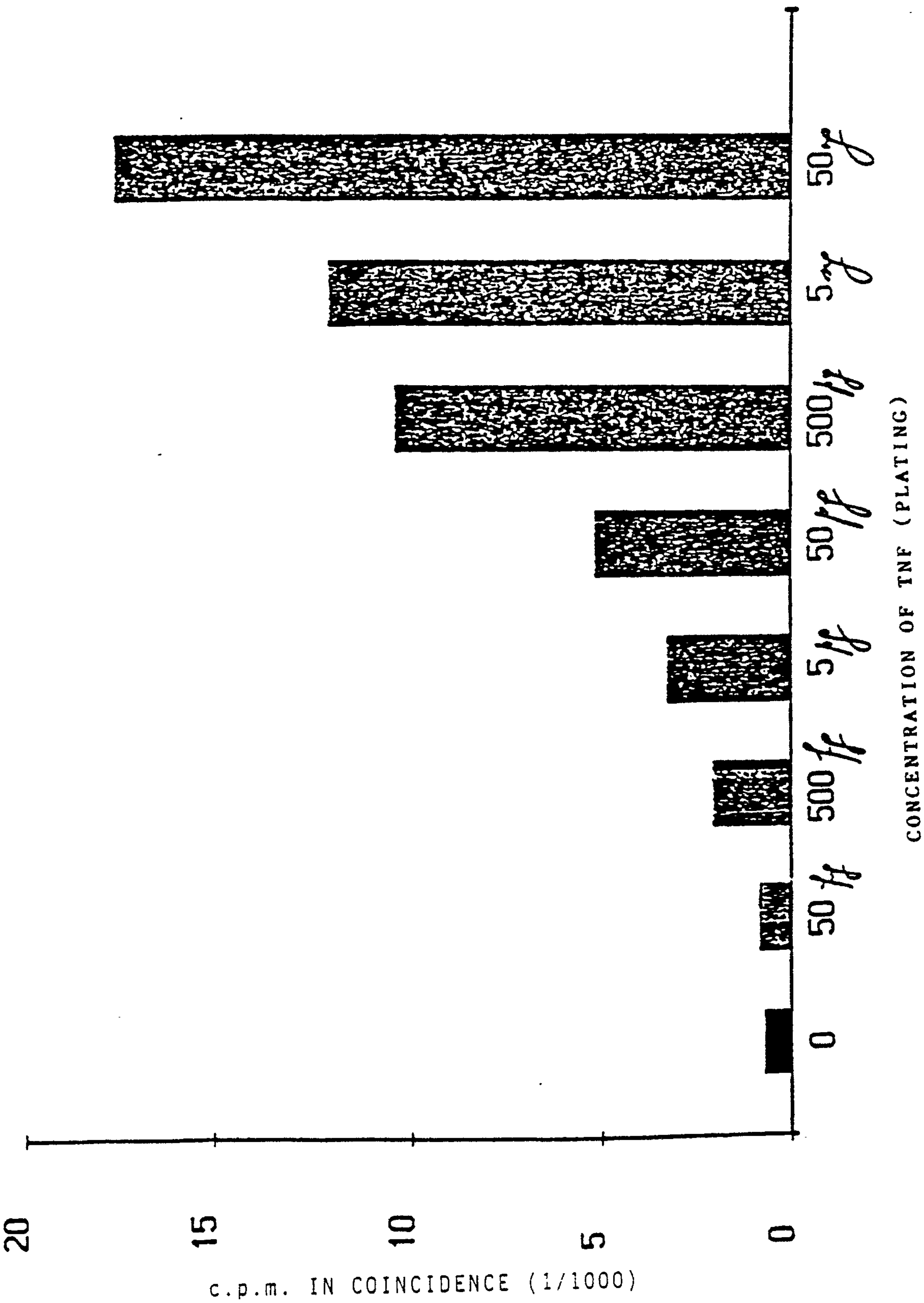
c.p.m. IN COINCIDENCE (1/1000)
0
5
10
15
20
CONCENTRATION OF TNF (PLATING)
0

FIGURE 7

DETECTION OF P-NITROPHENOL WITH

SPECTROPHOTOMETER

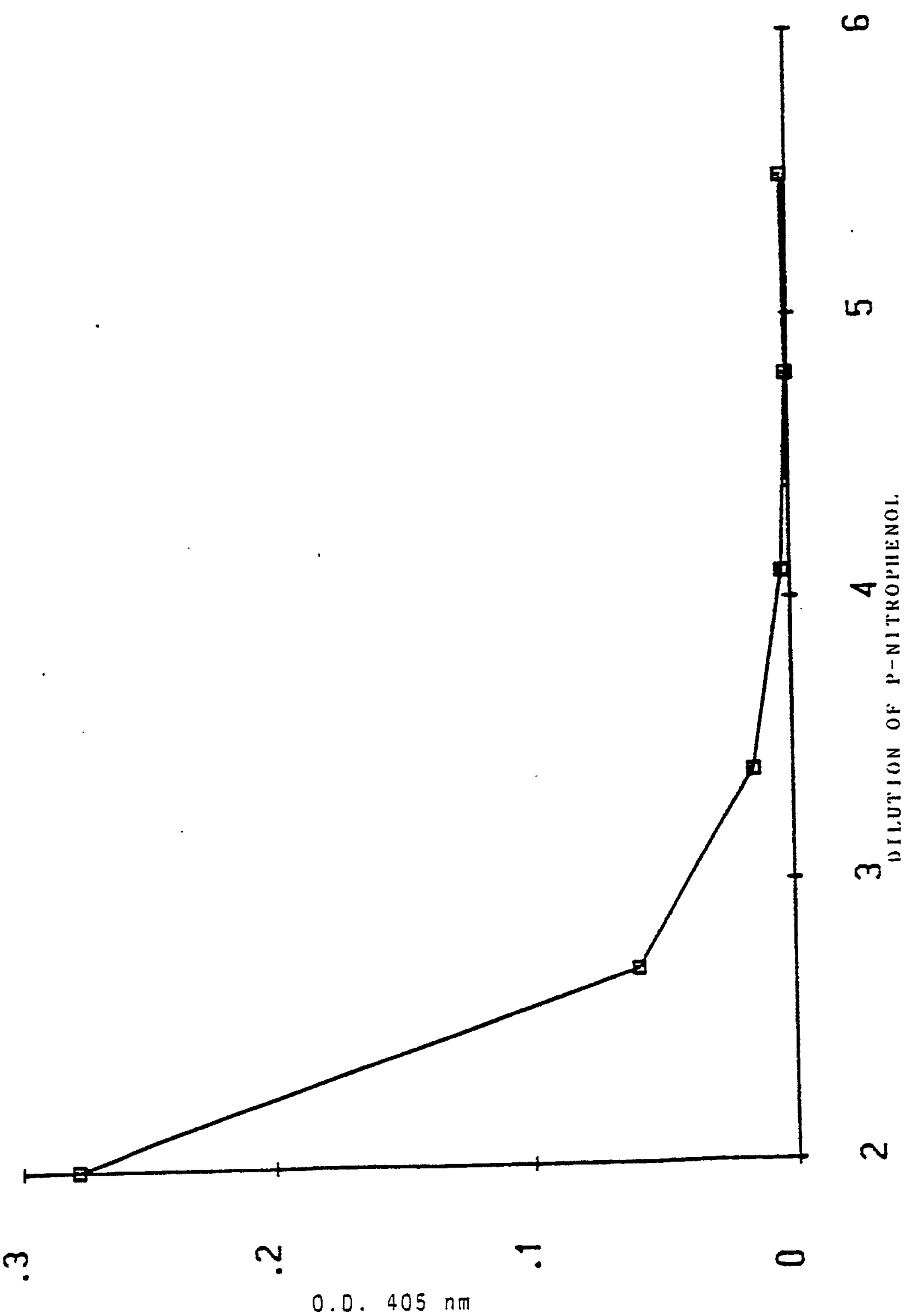

FIGURE 8

DETECTION OF P-NITROPHENOL

WITH LUCIGENIN CHEMILUMINESCENCE

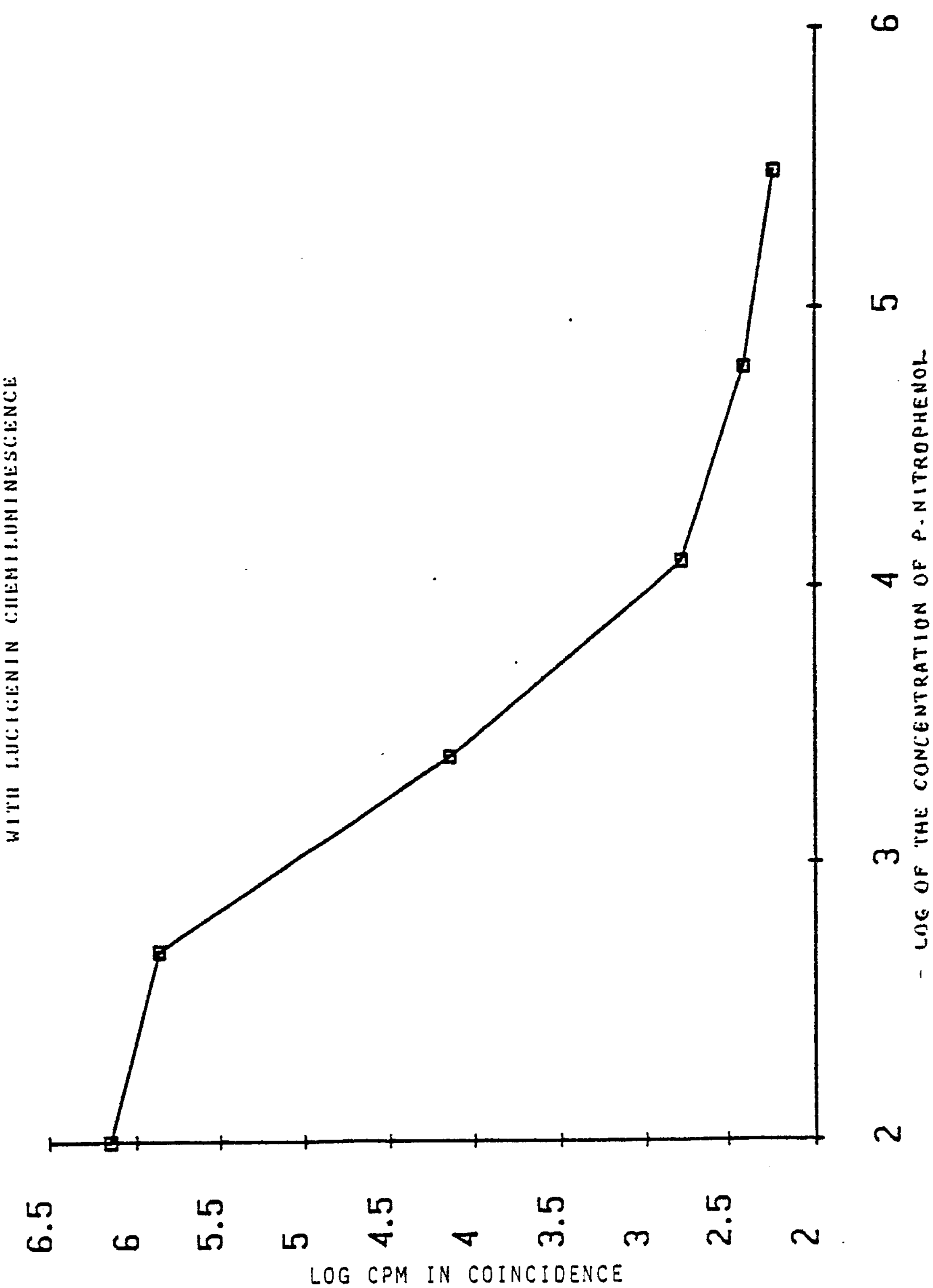

FIGURE 9

DETECTION OF GLYCINE.

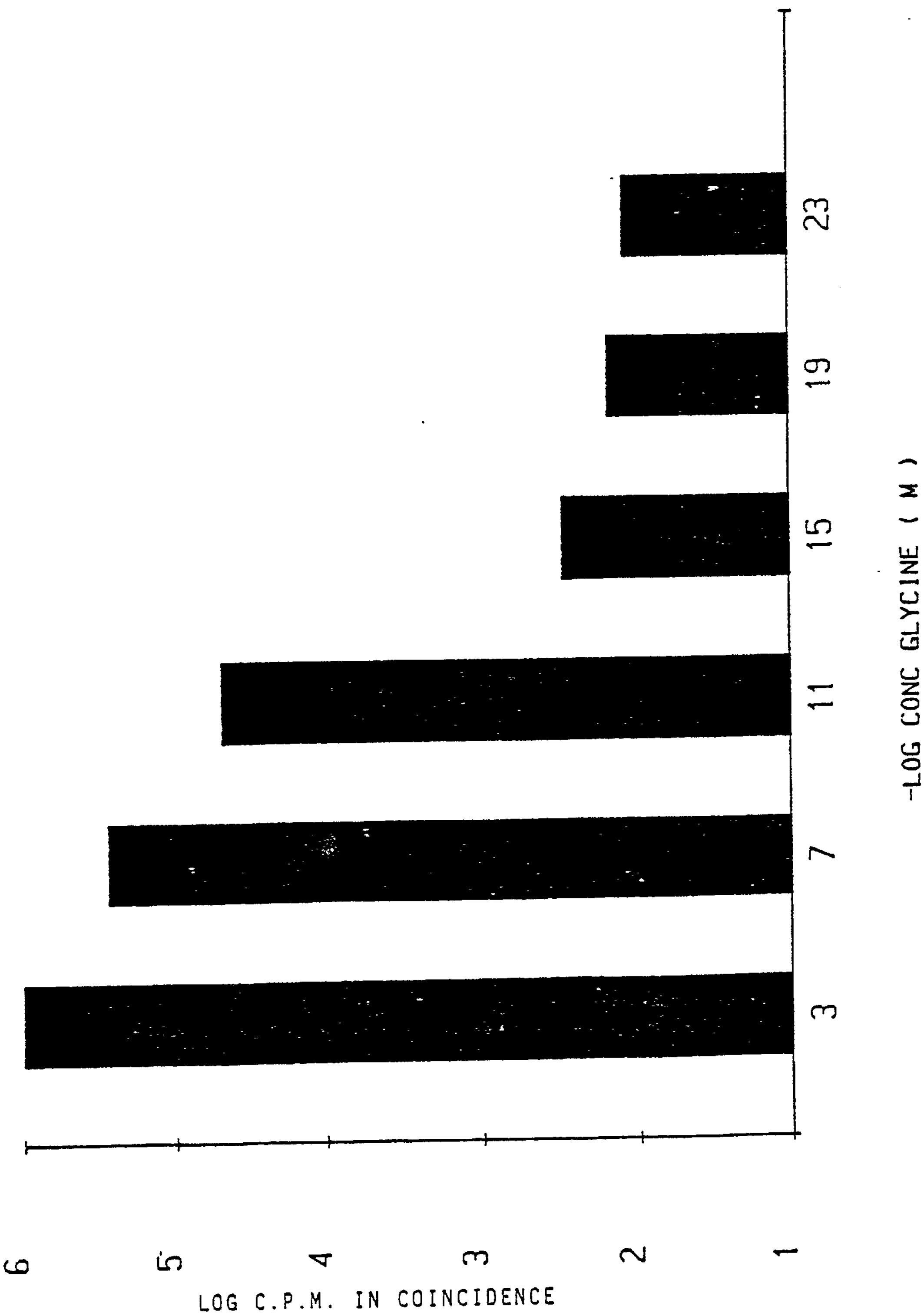

FIGURE 10
DNA-HYBRIDISATION

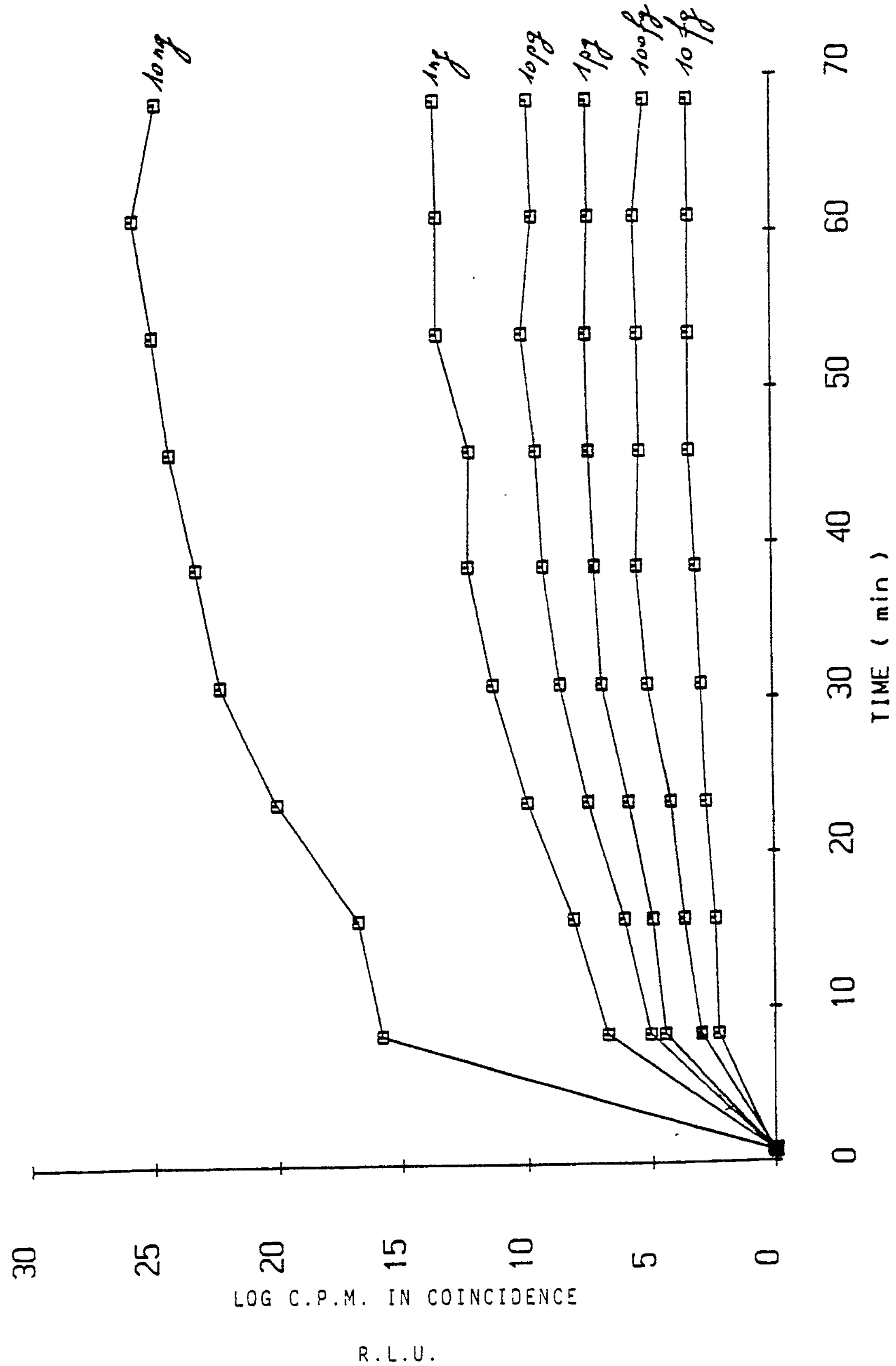

Figure 11

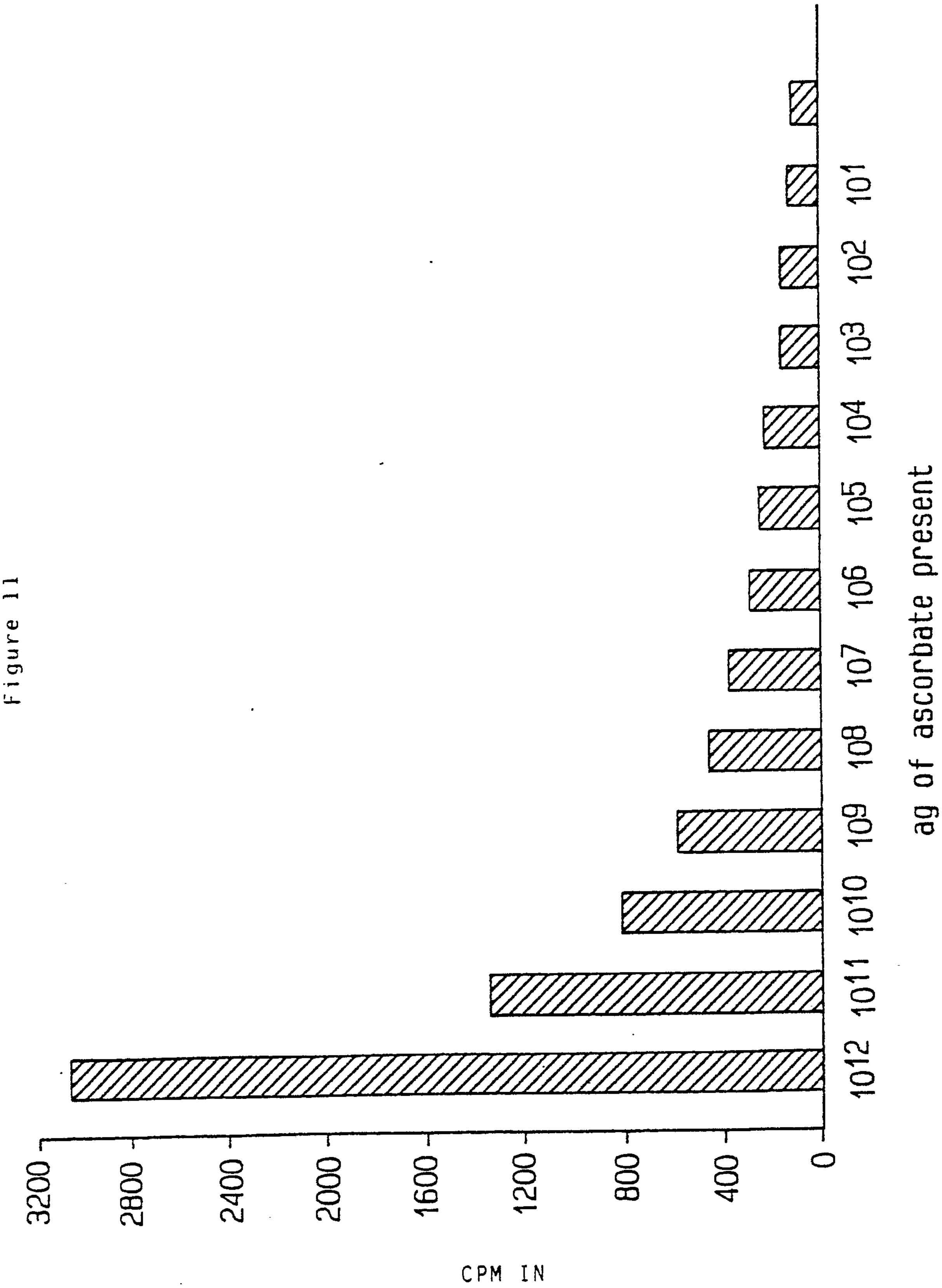
CPM IN
0
400
800
1200
1600
2000
2400
2800
3200
$10^{12}$
$10^{11}$
$10^{10}$
$10^{9}$
$10^{8}$
$10^{7}$
$10^{6}$
$10^{5}$
$10^{4}$
$10^{3}$
$10^{2}$
$10^{1}$
ag of ascorbate present

European Patent Office

# EUROPEAN SEARCH REPORT

Application Number

EP 90 40 2036

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 93, no. 16, 20th October 1980, page 701, abstract no. 160640g, Columbus, Ohio, US; L.I. DUBOVENKO et al.: "Determination of nickel and lead traces by a chemiluminescent method in a water-alcohol medium", & UKR. KHIM. ZH. (RUSS. ED.) 1980, 46(8), 854-7 | 1-3,9, 11,16 | G 01 N 33/52<br>G 01 N 31/22<br>C 12 Q 1/42<br>C 12 Q 1/68<br>G 01 N 33/68<br>G 01 N 33/543<br>G 01 N 33/58<br>G 01 N 33/577<br>C 12 Q 1/58 |
| X | CHEMICAL ABSTRACTS, vol. 84, no. 6, 9th February 1976, page 527, abstract no. 38257m, Columbus, Ohio, US; L.I. DUBOVENKO et al.: "Use of the chemiluminescence of lucigenin in an aqueous-alcohol solution for the determination of chromium", & IZV. VYSSH. UCHEBN. ZAVED., KHIM. KHIM. TEKHNOL. 1975, 18(8), 1211-13 | 1-3,9, 11,16 | |
| X | ANALYTICAL CHEMISTRY, vol. 51, no. 13, November 1979, pages 2092-2096; R.L. VEAZEY et al.: "Chemiluminescent determination of clinically important organic reductants" | 16,18 | TECHNICAL FIELDS SEARCHED (Int. Cl.5) |
| D,A | EP-A-0 263 657 (CIBA CORNING DIAGNOSTICS CORP.) | | G 01 N<br>C 12 Q |

The present search report has been drawn up for all claims

| Place of search | Date of completion of the search | Examiner |
|---|---|---|
| THE HAGUE | 27-08-1990 | CARTAGENA Y ABELLA P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document